# EUROPEAN PATENT APPLICATION

(11) **EP 1 236 470 A2**
(43) Date of publication of application: **04.09.2002**
(21) Application number: 02009992.5
(22) Date of filing: 23.11.1992
(51) Int. Cl.: A61K 31/565, A61K 31/56, A61K 31/57, C07J 3/00, C07J 5/00, C07J 9/00, C07J 11/00, C07J 41/00, C07J 51/00

(54) **Angiostatic steroids**

(30) Priority: 08.09.1992 US 941485; 02.06.1992 US 892448; 22.11.1991 US 796169
(62) Divisional of application: 93900609.4
(71) Applicant: Alcon Laboratories, Inc., Fort Worth, TX 76134 (US)
(72) Inventor: Clark, Abbot F., Arlington, TX 76017 (US); Conrow, Raymond E., Fort Worth, TX 76133 (US)
(74) Representative: Best, Michael, Dr.

(57) **Abstract**

Angiostatic steroids for use in controlling neovascularization and ocular hypertension are disclosed. Pharmaceutical compositions of the angiostatic steroids and methods for their use in treating neovascularization and ocular hypertension, including controlling the ocular hypertension associated with primary open angle glaucoma, are disclosed. In addition, the combination of the compounds with glucocorticoids for the prevention of elevated intraocular pressure during the treatment of inflammation is disclosed.

## Description

### Background of the Invention

### Field of the Invention

This invention relates to angiostatic steroids for controlling ocular hypertension. The compounds are also useful in preventing and treating neovascularization. Specifically, the invention is directed to new angiostatic steroids, pharmaceutical compositions comprising the angiostatic steroids, and methods of treatment which comprise administering these compositions to treat ocular hypertension, including controlling ocular hypertension associated with primary open angle glaucoma, and to treat neovascularization. In addition, the compounds can be used in combination with glucocorticoids to treat ocular inflammation without the significant intraocular pressure rise commonly associated with the use of glucocorticoids.

### Description of Related Art

Steroids functioning to inhibit angiogenesis in the presence of heparin or specific heparin fragments are disclosed in Crum, et al., *A New Class of Steroids Inhibits Angiogenesis in the Presence of Heparin or a Heparin Fragment,* Science, Vol.230, pp.1375-1378 (December 20, 1985). The authors refer to such steroids as "angiostatic" steroids. Included within the new class of steroids found to be angiostatic are the dihydro and tetrahydro metabolites of cortisol and cortexolone. In a follow-up study directed to testing a hypothesis as to the mechanism by which the steroids inhibit angiogenesis, it was shown that heparin/angiostatic steroid compositions cause dissolution of the basement membrane scaffolding to which anchorage dependent endothelia are attached resulting in capillary involution; see, Ingber, et al., *A Possible Mechanism for Inhibition of Angiogenesis by Angiostatic Steroids: Induction of Capillary Basement Membrane Dissolution,* Endocrinology Vol. 119, pp. 1768-1775 1986).

A group of tetrahydro steroids useful in inhibiting angiogenesis is disclosed in International Patent Application No. PCT/US86/02189, Aristoff, et al., (The Upjohn Company). The compounds are disclosed for use in treating head trauma, spinal trauma, septic or traumatic shock, stroke and hemorrhage shock. In addition, the patent application discusses the utility of these compounds in embryo implantation and in the treatment of cancer, arthritis and arteriosclerosis. Some of the steroids disclosed in Aristoff et al. are disclosed in U.S. Patent No. 4,771,042 in combination with heparin or a heparin fragment for inhibiting angiogenesis in a warm blooded animal.

Compositions of hydrocortisone, "tetrahydrocortisol-S," and U-72,745G, each in combination with a beta cyclodextrin, have been shown to inhibit corneal neovascularization: Li, et al., *Angiostatic Steroids Potentiated by Sulphated Cyclodextrin Inhibit Corneal Neovascularization,* Investigative Ophthalmology and Visual Science, Vol. 32, No. 11, pp. 2898-2905 (October, 1991). The steroids alone reduce neovascularization somewhat but are not effective alone in effecting regression of neovascularization.

Tetrahydrocortisol (THF) has been disclosed for its use in lowering the intraocular pressure (IOP) of rabbits made hypertensive with dexamethasone alone, or with dexamethasone/5-beta-dihydrocortisol; see Southren, et al., *Intraocular Hypotensive Effect of a Topically Applied Cortisol Metabolite: 3-alpha, 5-beta-tetrahydrocortisol*, Investigative Ophthalmology and Visual Science, Vol.28 (May, 1987). The authors suggest THF may be useful as an antiglaucoma agent. In U.S. Patent No. 4,863,912, issued to Southren et al. on September 5, 1989, pharmaceutical compositions containing THF and a method for using these compositions to control intraocular pressure are disclosed. THF has been disclosed as an angiostatic steroid in Folkman, et al., *Angiostatic Steroids,* Ann. Surg., Vol.206, No.3 (1987) wherein it is suggested angiostatic steroids may have potential use for diseases dominated by abnormal neovascularization, including diabetic retinopathy, neovascular glaucoma and retrolental fibroplasia.

Many compounds classified as glucocorticoids, such as dexamethasone and prednisolone, are very effective in the treatment of inflammed tissues; however, when these compounds are topically applied to the eye to treat ocular inflammation, certain patients experience elevated intraocular pressure. Patients who experience these elevations when treated with glucocorticoids are generally referred to as "steroid responders." These pressure elevations are of particular concern to patients who already suffer from elevated intraocular pressures, such as glaucoma patients. In addition, there is always a risk that the use of glucocorticoids in patients having normal intraocular pressures will cause pressure rises great enough to damage ocular tissues. Since glucocorticoid therapy is frequently long term (i.e., several days or more), there is potential for significant damage to ocular tissue as a result of prolonged elevations in intraocular pressure attributable to that therapy.

The following articles may be referenced for further background information concerning the well-recognized association between ophthalmic glucocorticoid therapy and elevations in intraocular pressure:
Kitazawa, *Increased Intraocular Pressure Induced by Corticosteroids*, Am. J. Ophthal., Vol.82 pp.492-493 (1976);
Cantrill, et al., *Comparison of In Vitro Potency of Corticosteroids with Ability to Raise Intraocular Pressure,* Am. J. Ophthal., Vol.79 pp.1012-1016 (1975); and
Mindel, et al., *Comparative Ocular Pressure Elevation by Medrysone, Fluorometholone, and Dexamethasone Phosphate,* Arch. Ophthal., Vol.98 pp.1577-1578 (1980).

Commonly assigned U.S. Application Serial No. 07/399,351 discloses the use of the angiostatic steroid tetrahydrocortexolone in combination with a glucocorticoid to treat ocular inflammation without the intraocular pressure elevating effect commonly associated with topical administration of glucocorticoids. In addition, commonly assigned International Application No. PCT/US90/04071 discloses the angiostatic steroids of Aristoff, et al. in combination with glucocorticoids to treat ocular inflammation without significant increase in intraocular pressure.

### Summary of the Invention

This invention is directed to angiostatic steroids and methods of using compositions of these steroids in inhibiting neovascularization. The compositions containing the steroids can be used for treatment of angiogenesis dependent diseases, for example: head trauma, spinal trauma, septic or traumatic shock, stroke, hemorrhagic shock, cancer, arthritis, arteriosclerosis, angiofibroma, arteriovenous malformations, corneal graft neovascularization, delayed wound healing, diabetic retinopathy, granulations, burns, hemangioma, hemophilic joints, hypertrophic scars, neovascular glaucoma, nonunion fractures, Osler-Weber Syndrome, psoriasis, pyogenic granuloma, retrolental fibroplasia, pterigium, scleroderma, trachoma, vascular adhesions, and solid tumor growth. In particular, the angiostatic steroids and compositions thereof are useful for controlling ocular neovascularization.

The invention also encompasses methods for controlling ocular hypertension and glaucoma through the systemic or local administration of the compositions disclosed herein.

The present invention also includes the use of the angiostatic steroids in combination with glucocorticoids for the treatment of ocular inflammation. The addition of at least one angiostatic steroid makes it possible to employ the potent antiinflammatory glucocorticoids without producing significant elevations in intraocular pressure.

### Brief Description of the Drawing

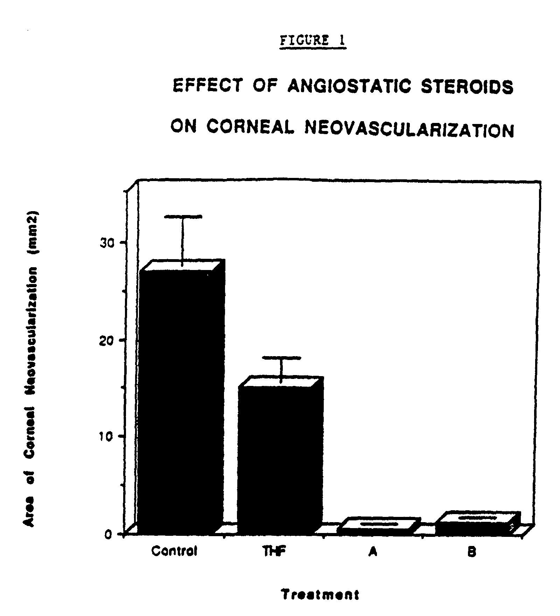

Figure 1 compares the ability of angiostatic steriods to inhibit neovascularization in the rabbit cornea.

### Detailed Description of Preferred Embodiments

The development of blood vessels for the purpose of sustaining viable tissue is known as angiogenesis or neovascularization. Agents which inhibit neovascularization are known by a variety of terms such as angiostatic, angiolytic or angiotropic agents. For purposes of this specification, the term "angiostatic agent" means compounds which can be used to control, prevent, or inhibit angiogenesis.

The angiostatic agents of the present invention are steroids or steroid metabolites. For purposes herein, the term "angiostatic steroids" means steroids and steroid metabolites which inhibit angiogenesis.

There is currently no effective method for controlling the neovascularization in anglogenesis-dependent diseases. In particular, ocular neovascularization has not been successfully treated in the past. Neovascularization of tissues in the front of the eye (i.e. the cornea, iris, and the trabecular meshwork) and other conditions, including conditions in the back of the eye, for example, retinal, subretinal, macular, and optical nerve head neovascularization, can be prevented and treated by administration of the steroids of this invention. The angiostatic steroids of the present invention are useful in preventing and treating neovascularization, including providing for the regression of neovascularization.

The angiostatic steroids can also be used for the control of ocular hypertension. In particular, the agents can be used for the treatment of primary open angle glaucoma.

The angiostatic steroids of the present invention have the following formula: 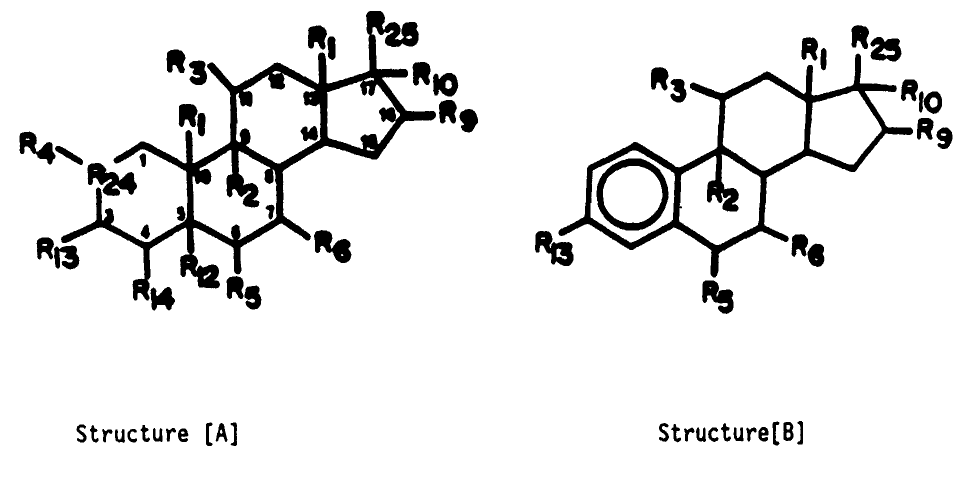 wherein R₁ is H, β-CH₃ or β-C₂H₅;
R₂ is F, C₉-C₁₁ double bond, C₉-C₁₁ epoxy, H or Cl;
R₃ is H, OR₂₆, OC(=O)R₂₇, halogen, C₉-C₁₁ double bond, C₉-C₁₁ epoxy, =O, -OH, -O-alkyl(C₁-C₁₂), -OC(=O)alkyl(C₁-C₁₂), -OC(=O)ARYL, -OC(=O)N(R)₂ or
-OC(=O)OR₇, wherein ARYL is furyl, thienyl, pyrrolyl, or pyridyl and each of said moieties is optionally substituted with one or two (C₁-C₄)alkyl groups, or ARYL is -(CH₂)_{f}-phenyl wherein f is 0 to 2 and the phenyl ring is optionally substituted with 1 to 3 groups selected from chlorine, fluorine, bromine, alkyl(C₁-C₃), alkoxy(C₁-C₃), thioalkoxy-(C₁-C₃), Cl₃C-, F₃C-, -NH₂ and -NHCOCH₃ and R is hydrogen, alkyl (C₁-C₄), or phenyl and each R can be the same or different, and R₇ is ARYL as herein defined, or alkyl(C₁-C₁₂);
R₄ is H, CH₃, Cl or F;
R₅ is H, OH, F, Cl, Br, CH₃, phenyl, vinyl or allyl;
R₆ is H or CH₃;
R₉ is CH₂CH₂OR₂₆, CH₂CH₂OC(=O)R₂₇, H, OH, CH₃, F, =CH₂, CH₂C(=O)OR₂₈, OR₂₆, O(C=O)R₂₇ or O(C=O)CH₂(C=O)OR₂₆
R₁₀ is -C≡CH, -CH=CH₂, halogen, CN, N₃, OR₂₆, OC(=O)R₂₇, H, OH, CH₃ or R₁₀ forms a second bond between positions C-16 and C-17;
R₁₂ is H or forms a double bond with R₁ or R₁₄;
R₁₃ is halogen, OR₂₆, OC(=O)R₂₇, NH₂, NHR₂₆, NHC(=O)R₂₇, N(R₂₆)₂, NC(=O)R₂₇, N₃, H, -OH, =O, -O-P(=O)(OH)₂, or -O-C(=O)-(CH₂)ₜCOOH where t is an integer from 2 to 6;
R₁₄ is H or forms a double bond with R₁₂;
R₁₅ is H, =O or -OH;
and R₂₃ with R₁₀ forms a cyclic phosphate;
wherein R₉ and R₁₅ have the meaning defined above;
or wherein R₂₃ is -OH, O-C(=O)-R₁₁, -OP(O)-(OH)₂, or -O-C(=O)-(CH₂)ₜCOOH wherein t is an integer from 2 to 6; and R₁₁ is -Y-(CH₂)ₙ-X-(CH₂)ₘ-SO₃H, -Y'-(CH₂)ₚ-X'-(CH₂)_{q}-NR₁₆R₁₇ or -Z(CH₂)ᵣQ,
wherein Y is a bond or -O-; Y' is a bond, -O-, or -S-; each of X and X' is a bond, -CON(R₁₈)-, -N(R₁₈)CO-, -O-, -S-, -S(O)-, or -S(O₂)-; R₁₈ is hydrogen or alkyl (C₁-C₄); each of R₁₆ and R₁₇ is a lower alkyl group of from 1 to 4 carbon atoms optionally substituted with one hydroxyl or R₁₆ and R₁₇ taken together with the nitrogen atom to which each is attached forms a monocyclic heterocycle selected from pyrrolidino, piperidino, morpholino, thiomorpholino, piperazino or N(lower)alkyl-piperazino wherein alkyl has from 1 to 4 carbon atoms; n is an integer of from 4 to 9; m is an integer of from 1 to 5; p is an integer of from 2 to 9; q is an integer of from 1 to 5;
Z is a bond or -O-; r is an integer of from 2 to 9; and Q is one of the following:
(1) -R₁₉-CH₂COOH wherein R₁₉ is -S-, -S(O)-, -S(O)₂-, -SO₂N(R₂₀)-, or N(R₂₀)SO₂-; and R₂₀ is hydrogen or lower alkyl-(C₁-C₄); with the proviso that the total number of carbon atoms in R₂₀ and (CH₂)ᵣ is not greater than 10; or
(2) -CO-COOH; or
(3) CON(R₂₁)CH(R₂₂)COOH wherein R₂₁ is H and R₂₂ is H, CH₃, -CH₂COOH, -CH₂CH₂COOH, -CH₂OH, -CH₂SH, -CH₂CH₂SCH₃, or
   -CH₂Ph-OH wherein Ph-OH is p-hydroxyphenyl;
   or R₂₁ is CH₃ and R₂₂ is H;
   or R₂₁ and R₂₂ taken together are -CH₂CH₂CH₂-;
   or -N(R₂₁)CH(R₂₂)COOH taken together is -NHCH₂CONHCH₂COOH; and pharmaceutically acceptable salts thereof;
with the proviso that except for the compound wherein R₁ is β-CH₃, R₂ and R₃ taken together form a double bond between positions 9 and 11, R₄ and R₆ are hydrogen, R₁₂ and R₁₄ taken together form a double bond between positions 4 and 5, R₅ is α-F, R₉ is β-CH₃, R₁₀ is α-OH, R₁₃ and R₁₅ are =O and R₂₃ is -OP(O)-(OH)₂, R₁₃ is =O only when R₂₃ with R₁₀ forms the above described cyclic phosphate.
- R₂₄ =: C, C₁-C₂ double bond, 0;
- R₂₅ =: C(R₁₅)CH₂-R₂₃, OH, OR₂₆, OC(=O)R₂₇, R₂₆, COOH, C(=O)OR₂₆, CHOHCH₂OH, CHOHCH₂OR₂₆, CHOHCH₂OC(=O)R₂₇, CH₂CH₂OH, CH₂CH₂OR₂₆, CH₂CH₂OC(=O)R₂₇, CH₂CN, CH₂N₃, CH₂NH₂, CH₂NHR₂₆, CH₂N(R₂₆)₂, CH₂OH, CH₂OR₂₆, CH₂O(C=O)R₂₇, CH₂O(P=O) (OH)₂, CH₂O(P=O) (OR₂₆)₂, CH₂SH, CH₂S-R₂₆, CH₂SC(=O)R₂₇, CH₂NC(=O)R₂₇, C(=O)CHR₂₈OH, C(=O)CHR₂₈OR₂₆, C(=O)CHR₂₈OC(=O)R₂₇ or R₁₀ and R₂₅ taken together may be =C(R₂₈)₂, that is, an optionally alkyl substituted methylene group;
wherein R₂₆ = C₁-C₆ (alkyl, branched alkyl, cycloalkyl, haloalkyl, aralkyl, aryl); R₂₇ = R₂₆ + OR₂₆; R₂₈ = H, C1-C6 (alkyl, branched alkyl, cycloalkyl).
Excepted from the compounds of Structure [A] are the compounds wherein R₁ is β-CH₃ or β-C₂H₅;
R₂ is H or Cl;
R₃ is H, =O, -OH, -O-alkyl(C₁-C₁₂), -OC(=O)alkyl(C₁-C₁₂), -OC(=O)ARYL, -OC(=O)N(R)₂ or α-OC(=O)OR₇, wherein ARYL is furyl, thienyl, pyrrolyl, or pyridyl and each of said moieties is optionally substituted with one or two (C₁-C₄)alkyl groups, or ARYL is -(CH₂)_{f}-phenyl wherein f is 0 to 2 and the phenyl ring is optionally substituted with 1 to 3 groups selected from chlorine, fluorine, bromine, alkyl(C₁-C₃), alkoxy(C₁-C₃), thioalkoxy-(C₁-C₃), Cl₃C-, F₃C-, -NH₂ and -NHCOCH₃ and R is hydrogen, alkyl (C₁-C₄), or phenyl and each R can be the same or different, and R₇ is ARYL as herein defined, or alkyl(C₁-C₁₂);
or
wherein R₂ and R₃ taken together are oxygen (-O-) bridging positions C-9 and C-11; or
wherein R₂ and R₃ taken together form a double bond between positions C-9 and C-11;
or R₂ is α-F and R₃ is β-OH;
or R₂ is α-Cl and R₃ is β-Cl;
and R₄ is H, CH₃, Cl or F;
R₅ is H, OH, F, Cl, Br, CH₃, phenyl, vinyl or allyl;
R₆ is H or CH₃;
R₉ is H, OH, CH₃, F or =CH₂;
R₁₀ is H, OH, CH₃ or R₁₀ forms a second bond between positions C-16 and C-17;
R₁₂ is -H or forms a double bond with R₁₄;
R₁₃ is H, -OH, =O, -O-P(O)(OH)₂, or -O-C(=O)-(CH₂)ₜCOOH where t is an integer from 2 to 6;
R₁₄ is H or forms a double bond with R₁₂;
R₁₅ is =O or -OH;
and R₂₃ with R₁₀ forms a cyclic phosphate;
wherein R₉ and R₁₅ have the meaning defined above;
or wherein R₂₃ is -OH, O-C(=O)-R₁₁, -OP(O)-(OH)₂, or -O-C(=O)-(CH₂)ₜCOOH wherein t is an integer from 2 to 6; and R₁₁ is -Y-(CH₂)ₙ-X-(CH₂)ₘ-SO₃H,
-Y'-(CH₂)ₚ-X'-(CH₂)_{q}-NR₁₆R₁₇ or -Z(CH₂)ᵣQ, wherein Y is a bond or -O-; Y' is a bond, -O-, or -S-; each of X and X' is a bond, -CON(R₁₈)-, -N(R₁₈)CO-, -O-, -S-, -S(O)-, or -S(O₂)-; R₁₈ is hydrogen or alkyl (C₁-C₄); each of R₁₆ and R₁₇ is a lower alkyl group of from 1 to 4 carbon atoms optionally substituted with one hydroxyl or R₁₆ and R₁₇ taken together with the nitrogen atom to which each is attached forms a monocyclic heterocycle selected from pyrrolidino, piperidino, morpholino, thiomorpholino, piperazino or N(lower)alkyl-piperazino
wherein alkyl has from 1 to 4 carbon atoms; n is an integer of from 4 to 9; m is an integer of from 1 to 5; p is an integer of from 2 to 9; q is an integer of from 1 to 5;
Z is a bond or -O-; r is an integer of from 2 to 9; and Q is one of the following:
(1) -R₁₉-CH₂COOH wherein R₁₉ is -S-, -S(O)-, -S(O)₂-, -SO₂N(R₂₀)-, or N(R₂₀)SO₂-; and R₂₀ is hydrogen or lower alkyl-(C₁-C₄); with the proviso that the total number of carbon atoms in R₂₀ and (CH₂)ᵣ is not greater than 10; or
(2) -CO-COOH; or
(3) CON(R₂₁)CH(R₂₂)COOH wherein R₂₁ is H and R₂₂ is H, CH₃, -CH₂COOH, -CH₂CH₂COOH, -CH₂OH, -CH₂SH, -CH₂CH₂SCH₃, or
   -CH₂Ph-OH wherein Ph-OH is p-hydroxyphenyl;
   or R₂₁ is CH₃ and R₂₂ is H;
   or R₂₁ and R₂₂ taken together are -CH₂CH₂CH₂-;
   or -N(R₂₁)CH(R₂₂)COOH taken together is -NHCH₂CONHCH₂COOH; and pharmaceutically acceptable salts thereof;
with the proviso that except for the compound wherein R₁ is β-CH₃, R₂ and R₃ taken together form a double bond between positions 9 and 11, R₄ and R₆ are hydrogen, R₁₂ and R₁₄ taken together form a double bond between positions 4 and 5, R₅ is α-F, R₉ is β-CH₃, R₁₀ is α-OH, R₁₃ and R₁₅ are =O and R₂₃ is -OP(O)-(OH)₂, R₁₃ is =O only when R₂₃ with R₁₀ forms the above described cyclic phosphate.

Unless specified otherwise, all substituent groups attached to the cyclopentanophenanthrene moiety of Structures [A] and [B] may be in either the alpha or beta position. Additionally, the above structures include all pharmaceutically acceptable salts of the angiostatic steroids.

Preferred anglostatic steroids for the treatment of ocular hypertension, neovascular diseases and ocular inflammation are:

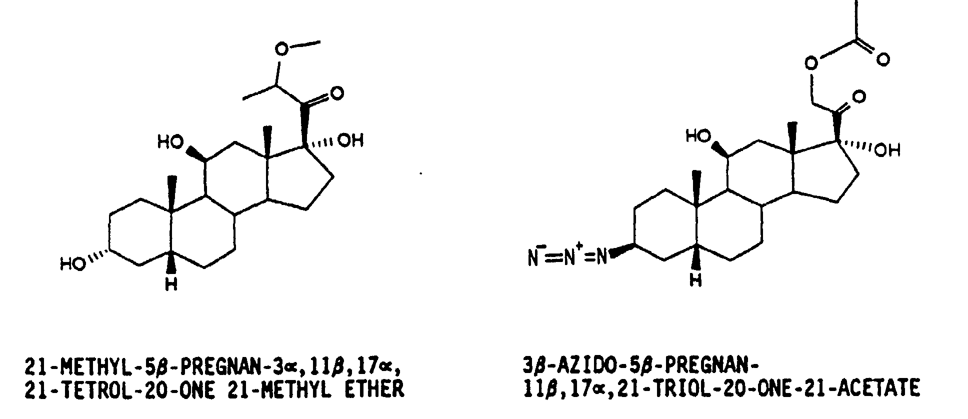

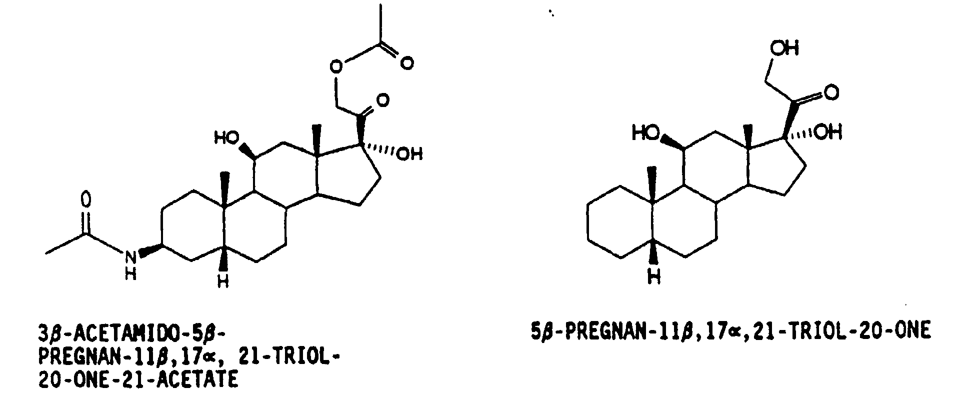

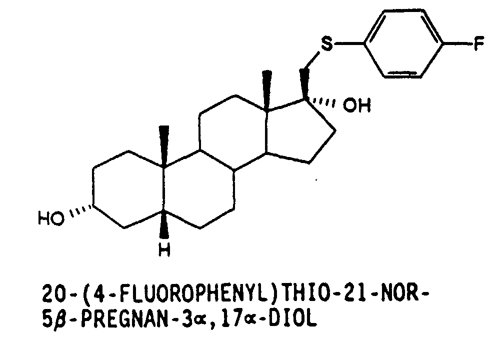

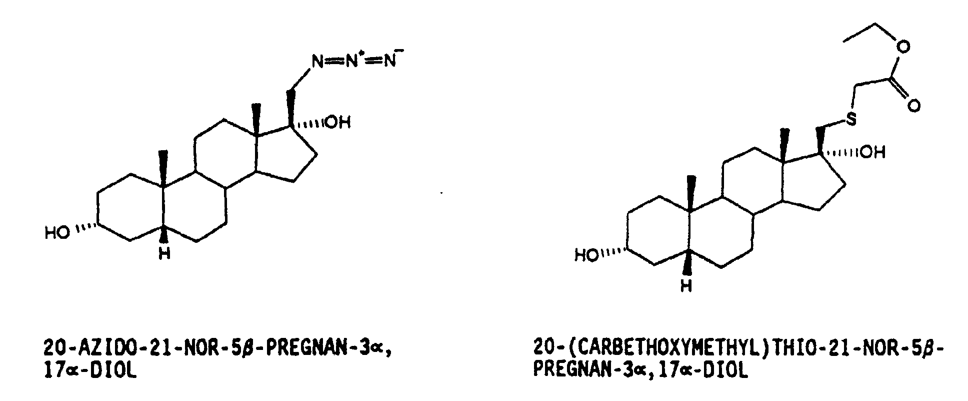

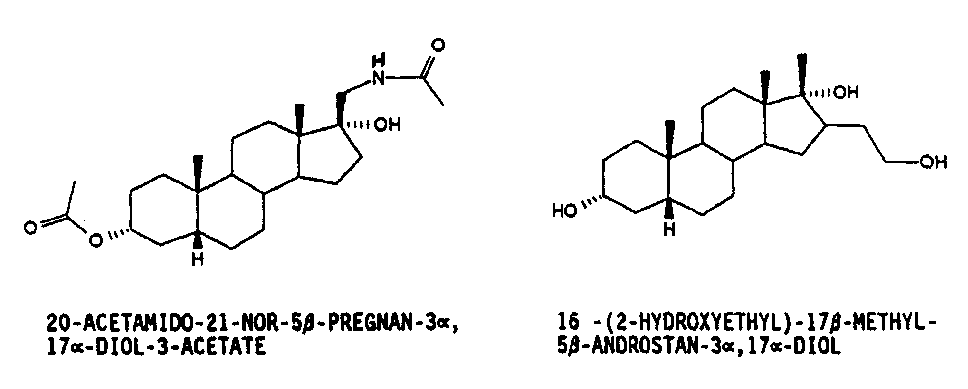

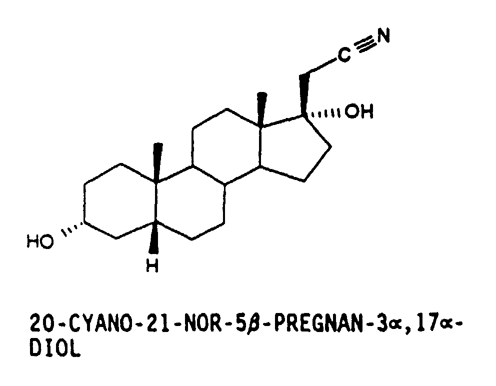

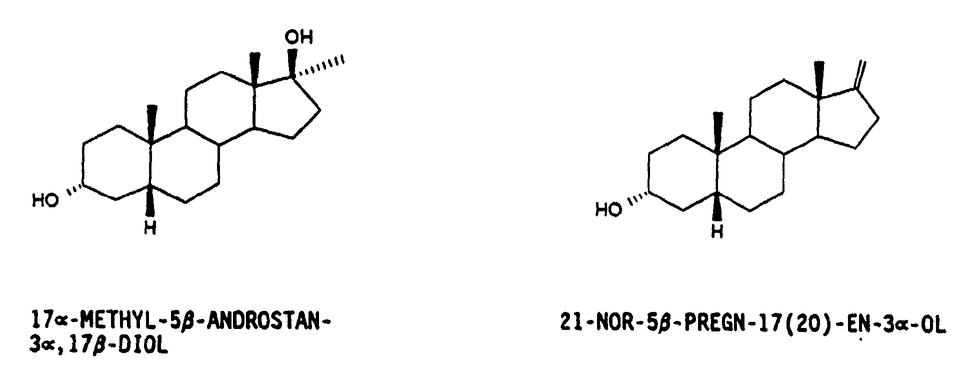

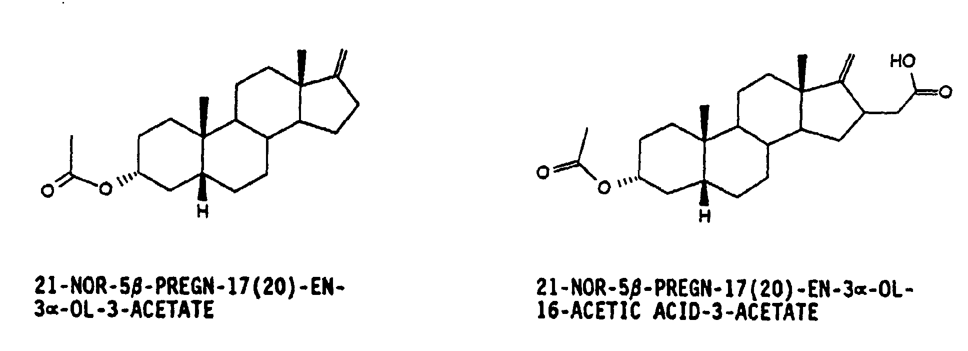

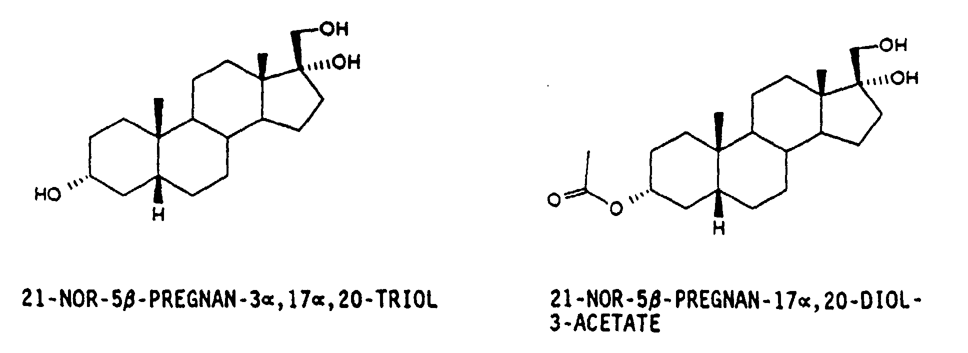

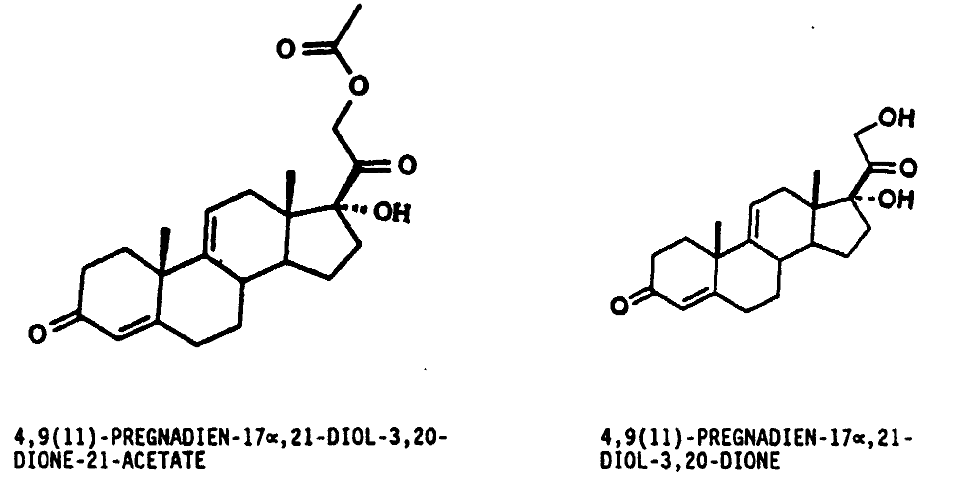

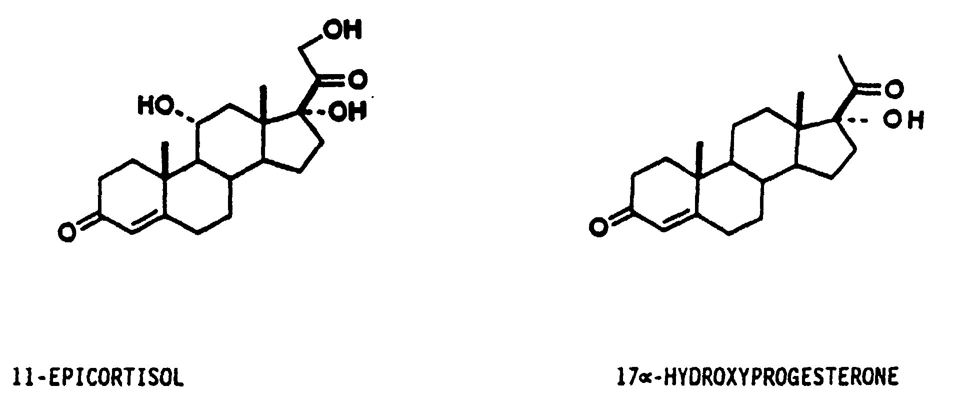

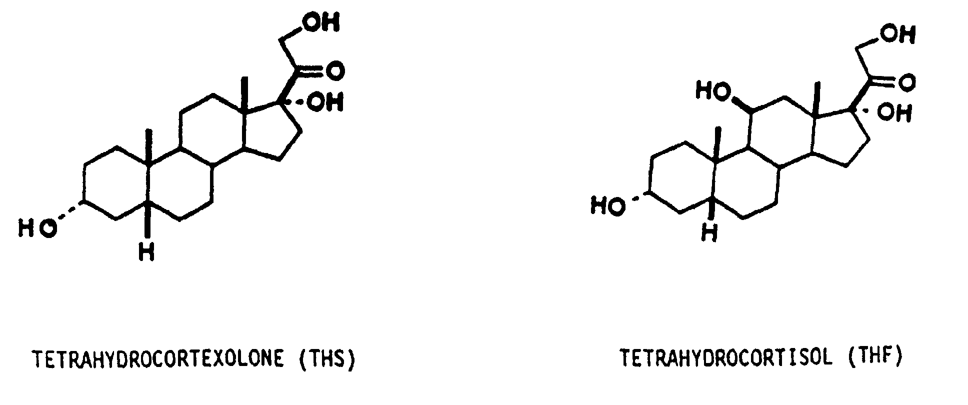

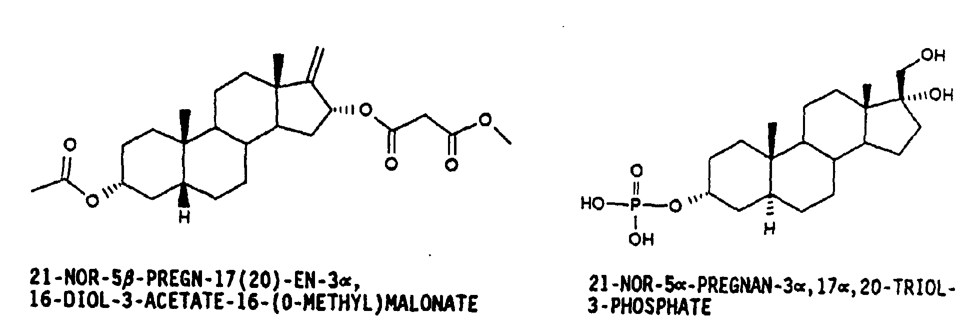

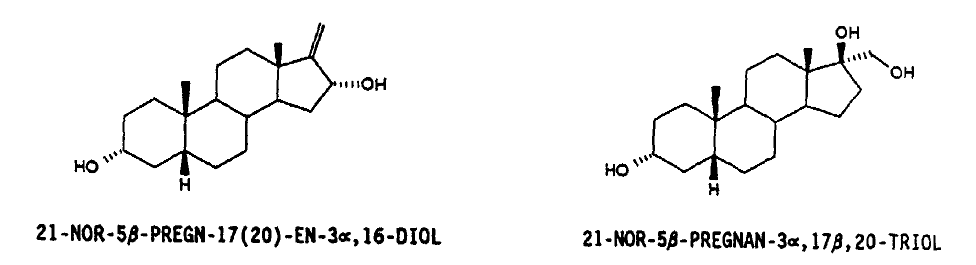

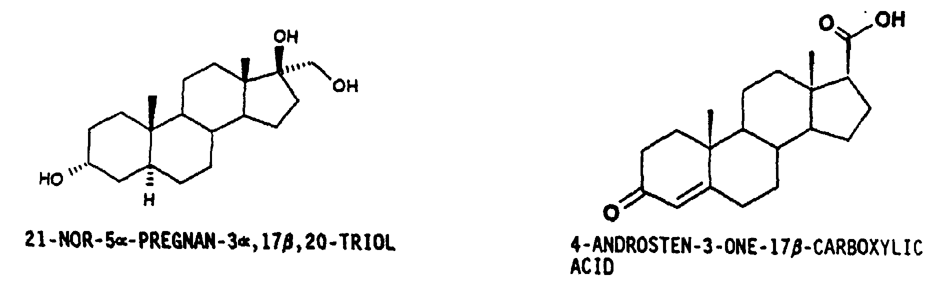

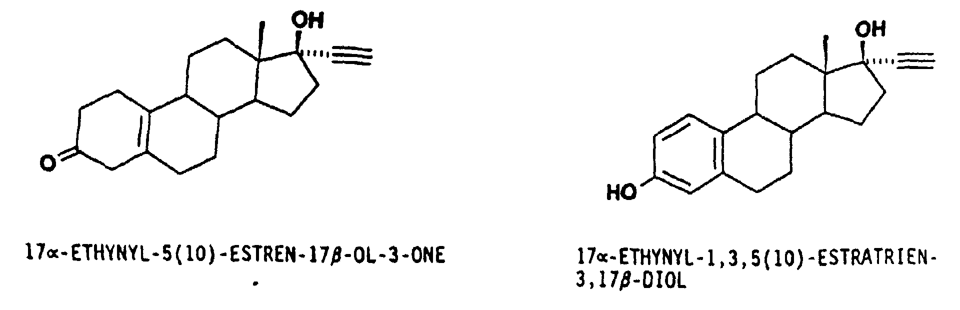

Most preferred compounds for preventing and treating neovascularization are:
4,9(11)-Pregnadien-17α,21-diol-3,20-dione-21-acetate
21-Nor-5β-pregn-17(20)-en-3α,16-diol-3-acetate-16-(O-methyl)malonate
4,9(11)-Pregnadien-17α,21-diol-3,20-dione
21-Nor-5β-pregnan-3α,17α,20-triol-3-acetate
21-Nor-5α-pregnan-3α,17α,20-triol-3-phosphate

The angiostatic steroids of the present invention are useful in inhibiting neovascularization and can be used in treating the neovascularization associated with: head trauma, spinal trauma, systemic or traumatic shock, stroke, hemorrhagic shock, cancer, arthritis, arteriosclerosis, angiofibroma, arteriovenous malformations, corneal graft neovascularization, delayed wound healing, diabetic retinopathy, granulations, burns, hemangioma, hemophilic joints, hypertrophic scars, neovascular glaucoma, nonunion fractures, Osler-Weber Syndrome, psoriasis, pyogenic granuloma, retrolental fibroplasia, pterigium, scleroderma, trachoma, vascular adhesions, and solid tumor growth.

In particular, the angiostatic steroids are useful in preventing and treating any ocular neovascularization, including, but not limited to: retinal diseases (diabetic retinopathy, chronic glaucoma, retinal detachment, sickle cell retinopathy, senile macular degeneration due to subretinal neovascularization); rubeosis iritis; inflammatory diseases; chronic uveitis; neoplasms (retinoblastoma, pseudoglioma); Fuchs' heterochromic iridocyclitis; neovascular glaucoma; corneal neovascularization (inflammatory, transplantation, developmental hypoplasia of the iris); neovascularization resulting following a combined vitrectomy and lensectomy; vascular diseases (retinal ischemia, choroidal vascular insufficiency, choroidal thrombosis, carotid artery ischemia); pterigium; neovascularization of the optic nerve; and neovascularization due to penetration of the eye or contusive ocular injury.

The initiation of new blood vessel formation may arise quite differently in various tissues or as a result of different diseases. Many substances have been found to induce neovascularization, see, Folkman, et al., *Angiogenic Factors,* Science, Volume 235, pp. 442-447 (1987). However, it is believed, that once initiated, the process of neovascularization is similar in all tissues regardless of the associated disease, Furcht, *Critical Factors Controlling Angiogenesis: Cell Products, Cell Matrix, and Growth Factors,* Laboratory Investigation, Volume 55, No. 5, pp. 505-509 (1986).

There are a variety of theories regarding the mechanism of action of angiostatic steroids. For example, angiostatic steroid induced inhibition of neovascularization may occur due to, dissolution of the capillary basement membrane, Ingber, et al., *Supra;* inhibition of vascular endothelial cell proliferation, Cariou, et al., *Inhibition of Human Endothelial Cell Proliferation by Heparin and Steroids,* Cell Biology International Reports, Vol. 12, No. 12, pp. 1037-1047 (December, 1988); effect on vascular endothelial cell laminin expression, Tokida, et al., *Production of Two Variant Laminin Forms by Endothelial Cells and Shift of Their Relative Levels by Angiostatic Steroids,* The Journal of Biological Chemistry, Vol. 264, No. 30, pp. 18123-18129 (October 25, 1990); inhibition of vascular cell collagen synthesis, Maragoudakis, et al., *Antiangiogenic Action of Heparin Plus Cortisone is Associated with Decreased Collagenous Protein Synthesis in the Chick Chorioallantolc Membrane System,* The Journal of Pharmacology and Experimental Therapeutics, Vol. 251, No. 2, pp. 679-682 (1989); and inhibition of vascular endothelial cell plasminogen activator activity, Ashino-Fuse, et al., *Medroxyprogesterone Acetate, An Anti-Cancer and Anti-Angiogenic Steroid, Inhibits the Plasminogen Activator in Bovine Endothelial Cells,* Int. J. Cancer, 44, pp. 859-864 (1989).

There are many theories associated with the cause of neovascularization, and there may be different inducers depending on the disease or surgery involved, BenEzra, *Neovasculogenic Ability of Prostaglandins, Growth Factors, and Synthetic Chemoattractants,* American Journal of Ophthalmology, Volume 86, No. 4, pp. 455-461, (October, 1978). Regardless of the cause or the associated disease or surgery, it is believed that angiostatic agents work by inhibiting one or more steps in the process of neovascularization. Therefore, the angiostatic steroids of this invention are useful in the treatment and prevention of neovascularization associated with a variety of diseases and surgical complications.

The angiostatic steroids of the present invention may be incorporated in various formulations for delivery. The type of formulation (topical or systemic) will depend on the site of disease and its severity. For administration to the eye, topical formulations can be used and can include ophthalmologically acceptable preservatives, surfactants, viscosity enhancers, buffers, sodium chloride, and water to form aqueous sterile ophthalmic solutions and suspensions. In order to prepare sterile ophthalmic ointment formulations, an angiostatic steroid is combined with a preservative in an appropriate vehicle, such as mineral oil, liquid lanolin, or white petrolatum. Sterile ophthalmic gel formulations comprising the angiostatic steroids of the present invention can be prepared by suspending an angiostatic steroid in a hydrophilic base prepared from a combination of, for example, Carbopol® (a carboxy vinyl polymer available from the BF Goodrich Company) according to published formulations for analogous ophthalmic preparations. Preservatives and antimicrobial agents may also be incorporated in such gel formulations. Systemic formulations for treating ocular neovascularization can also be used, for example, orally ingested tablets and formulations for intraocular and periocular injection.

The specific type of formulation selected will depend on various factors, such as the angiostatic steroid or its salt being used, the dosage frequency, and the location of the neovascularization being treated. Topical ophthalmic aqueous solutions, suspensions, ointments, and gels are the preferred dosage forms for the treatment of neovascularization in the front of the eye (the cornea, iris, trabecular meshwork); or neovascularization of the back of the eye if the angiostatic agent can be formulated such that it can be delivered topically and the agent is able to penetrate the tissues in the front of the eye. The angiostatic steroid will normally be contained in these formulations in an amount from about 0.01 to about 10.0 weight/percent. Preferable concentrations range from about 0.1 to about 5.0 weight/percent. Thus, for topical administration, these formulations are delivered to the surface of the eye one to six times a day, depending on the routine discretion of the skilled clinician. Systemic administration, for example, in the form of tablets is useful for the treatment of neovascularization particularly of the back of the eye, for example, the retina. Tablets containing 10-1000 mg of angiostatic agent can be taken 2-3 times per day depending on the discretion of the skilled clinician.

The preferred compounds for controlling ocular hypertension are: 21-Nor-5β-pregnan-3α,17α,20-triol;5β-pregnan-11β,17α,21-triol-20-one;4,9(11)-Pregnadien-17α,21-diol-3,20-dione-21-acetate, and 4,9(11)-Pregnadien-17α,21-diol-3,20-dione. The most preferred compound is 4,9(11)-Pregnadien-17α,21-diol-3,20-dione-21-acetate.

Without intending to be bound by any theory, it is believed that the angiostatic steroids of the type described above act to control intraocular pressure by inhibiting the accumulation or stimulating the dissolution of amorphous extracellular material in the trabecular meshwork of the eye. The presence of this amorphous extracellular material alters the integrity of the healthy trabecular meshwork and is a symptom associated with primary open angle glaucoma (POAG). It is not well understood why this amorphous extracellular material builds up in the trabecular meshwork of persons suffering from POAG. However, it has been found that the amorphous extracellular material is generally composed of glycosaminoglycans (GAGs) and basement membrane material; see, *Ophthalmology,* Vol.90, No.7 (July 1983); *Mayo Clin. Proc*, Vol.61, pp.59-67 (Jan.1986); and *Pediat. Neurosci.* Vol.12, pp.240-251 (1985-86). When these materials build up in the trabecular meshwork, the aqueous humor, normally present in the anterior chamber of the eye, cannot leave this chamber through its normal route (the trabecular meshwork) at its normal rate. Therefore, a normal volume of aqueous humor is produced by the ciliary processes of the eye and introduced into the anterior chamber, but its exit through the trabecular meshwork is abnormally slow. This results in a buildup of pressure in the eye, ocular hypertension, which can translate into pressure on the optic nerve. The ocular hypertension so generated can lead to blindness due to damage to the optic nerve.

Many methods for treating primary open angle glaucoma and ocular hypertension concentrate on blocking production of aqueous humor by the eye. However, aqueous humor is the fundamental source of nourishment for the tissues of the eye, particularly the cornea and lens which are not sustained by blood supply. Therefore, it is not desirable to deprive these tissues of the necessary irrigation and nutrition provided by the aqueous humor. It is desirable to strive for normal exit of the aqueous humor by maintaining the normal integrity of the trabecular meshwork. This is accomplished according to the present invention by the administration of angiostatic steroids.

It is believed that the angiostatic steroids disclosed herein function in the trabecular meshwork in a similar manner as shown by Ingber, et al., wherein it was shown that angiostatic steroids caused dissolution of the basement membrane scaffolding using a chick embryo neovascularization model; *Endocrinology,* 119, pp.1768-1775 (1986). It is believed that the angiostatic steroids of the present invention prevent the accumulation, or promote the dissolution of, amorphous extracellular materials in the trabecular meshwork by inhibiting the formation of basement membrane materials and glycosaminoglycans. Thus, by preventing the development of these materials or promoting their dissolution, the normal integrity of the trabecular meshwork is retained and aqueous humor may flow through the trabecular meshwork at normal rates. As a result, the intraocular pressure of the eye is controlled.

The angiostatic steroids of the present invention may be incorporated in various formulations for delivery to the eye to control ocular hypertension. For example, topical formulations can be used and can include ophthalmologically acceptable preservatives, surfactants, viscosity enhancers, buffers, sodium chloride and water to form aqueous sterile ophthalmic solutions and suspensions. In order to prepare sterile ophthalmic ointment formulations, an angiostatic steroid is combined with a preservative in an appropriate vehicle, such as mineral oil, liquid lanolin or white petrolatum. Sterile ophthalmic gel formulations comprising the angiostatic steroids of the present invention can be prepared by suspending an angiostatic steroid in a hydrophilic base prepared from a combination of, for example, Carbopol® 940 (a carboxyvinyl polymer available from the B.F. Goodrich Company) according to published formulations for analogous ophthalmic preparations. Preservatives and tonicity agents may also be incorporated in such gel formulations. The specific type of formulations selected will depend on various factors, such as the angiostatic steroid or its salt being used, and the dosage frequency. Topical ophthalmic aqueous solutions, suspensions, ointments and gels are the preferred dosage forms. The angiostatic steroid will normally be contained in these formulations in an amount of from about 0.005 to about 5.0 weight percent (wt.%). Preferable concentrations range from about 0.05 to about 2.0 wt.%. Thus, for topical administration, these formulations are delivered to the surface of the eye one to four times per day, depending upon the routine discretion of the skilled clinician.

In addition, antiinflammatory compositions of glucocorticoids can contain one or more angiostatic steroids of the present invention, preferably tetrahydrocortisol. These compositions will contain one or more glucocorticoids in an antiinflammatory effective amount and will contain one or more angiostatic steroids of the present invention in an amount effective to inhibit the IOP elevating effect of the glucocorticoids. The amount of each component will depend on various factors, such as the relative tendency of certain glucocorticoids to cause IOP elevations, the severity and type of ocular inflammation being treated, the estimated duration of the treatment, and so on. In general, the ratio of the amount of glucocorticoid to the amount of angiostatic steroid on a weight to weight basis will be in the range of 10:1 to 1:20. The concentration of the glucocorticoid component will typically be in the range of about 0.01% to about 2.0% by weight. The concentration of the angiostatic steroid component will typically be in the range of about 0.05% to about 5.0% by weight.

The above-described active ingredients may be incorporated into various types of systemic and ophthalmic formulations. For example, for topical ocular administration, the active ingredients may be combined with ophthalmologically acceptable preservatives, surfactants, viscosity enhancers, buffers, toxicity agents and water to form an aqueous, sterile ophthalmic suspension. In order to prepare sterile ophthalmic ointment formulations, the active ingredients are combined with a preservative in an appropriate vehicle, such as mineral oil, liquid lanolin, or white petrolatum. Sterile ophthalmic gel formulations may be prepared by suspending the active ingredient in a hydrophilic base prepared from the combination of Carbopol® 940 (a carboxy vinyl polymer available from the B.F. Goodrich Company) according to published formulations for analogous ophthalmic preparations; preservatives and tonicity agents can also be incorporated. The specific type of formulation selected will depend on various factors, such as the severity and type of ophthalmic inflammation being treated, and dosage frequency. Ophthalmic solutions, suspensions, ointments and gels are the preferred dosage forms, and topical application to the inflamed ocular tissue is the preferred route of administration.

The following examples illustrate formulations and synthesis of compounds of the present invention, but are in no way limiting.

### Example 1

The topical compositions are useful for controlling ocular hypertension or controlling ocular neovascularization.

| Component | wt.% |
|---|---|
| Angiostatic Steroid | 0.005-5.0 |
| | |
| Tyloxapol | 0.01-0.05 |
| HPMC | 0.5 |
| Benzalkonium Chloride | 0.01 |
| Sodium Chloride | 0.8 |
| Edetate Disodium | 0.01 |
| NaOH/HCl | q.s. pH 7.4 |
| Purified Water | q.s. 100 mL |

### Example 2

The composition is useful for controlling ocular hypertension.

| Component | wt.% |
|---|---|
| 21-Nor-5β-pregnan-3α,17α,20-triol | 1.0 |
| | |
| Tyloxapol | 0.01-0.05 |
| HPMC | 0.5 |
| Benzalkonium Chloride | 0.01 |
| Sodium Chloride | 0.8 |
| Edetate Disodium | 0.01 |
| NaOH/HCl | q.s. pH 7.4 |
| Purified Water | q.s. 100 mL |

The above formulation is prepared by first placing a portion of the purified water into a beaker and heating to 90°C. The hydroxypropylmethylcellulose (HPMC) is then added to the heated water and mixed by means of vigorous vortex stirring until all of the HPMC is dispersed. The resulting mixture is then allowed to cool while undergoing mixing in order to hydrate the HPMC. The resulting solution is then sterilized by means of autoclaving in a vessel having a liquid inlet and a hydrophobic, sterile air vent filter.

The sodium chloride and the edetate disodium are then added to a second portion of the purified water and dissolved. The benzalkonium chloride is then added to the solution, and the pH of the solution is adjusted to 7.4 with 0.1M NaOH/HCl. The solution is then sterilized by means of filtration.

21-Nor-5β-pregnan-3α,17α,20-triol is sterilized by either dry heat or ethylene oxide. If ethylene oxide sterilization is selected, aeration for at least 72 hours at 50°C. is necessary. The sterilized steroid is weighed aseptically and placed into a pressurized ballmill container. The tyloxapol, in sterilized aqueous solution form, is then added to the ballmill container. Sterilized glass balls are then added to the container and the contents of the container are milled aseptically at 225 rpm for 16 hours, or until all particles are in the range of approximately 5 microns.

Under aseptic conditions, the micronized drug suspension formed by means of the preceding step is then poured into the HPMC solution with mixing. The ballmill container and balls contained therein are then rinsed with a portion of the solution containing the sodium chloride, the edetate disodium and benzalkonium chloride. The rinse is then added aseptically to the HPMC solution. The final volume of the solution is then adjusted with purified water and, if necessary, the pH of the solution is adjusted to pH 7.4 with NaOH/HCl.

### Example 3

The following formulation is representative of the antiinflammatory compositions of the present invention.

| Component | wt.% |
|---|---|
| 4,9(11)Pregnadien-17α,21-diol-3,20-dione-21-acetate | 1.0 |
| | |
| Dexamethasone | 0.1 |
| Tyloxapol | 0.01 to 0.05 |
| HPMC | 0.5 |
| Benzalkonium Chloride | 0.01 |
| Sodium Chloride | 0.8 |
| Edetate Disodium | 0.01 |
| NaOH/HCl | q.s. pH 7.4 |
| Purified Water | q.s. 100 mL |

The above formulation is prepared in the same manner set forth in Example 2, sterilizing and adding the dexamethasone to the steroid before placing both into a pressurized ballmill container.

### Example 4

The following formulation is another example of the antiinflammatory compositions of the present invention.

| | wt.% |
|---|---|
| Tetrahydrocortisol | 1.0 |
| Prednisolone Acetate | 1.0 |
| Tyloxapol | 0.01 to 0.05 |
| HPMC | 0.5 |
| Benzalkonium Chloride | 0.01 |
| Sodium Chloride | 0.8 |
| Edetate Disodium | 0.01 |
| NaOH/HCl | q.s. pH 7.4 |
| Purified Water | q.s. 100 mls |

The above formulation is prepared in the same manner set forth in Example 2, sterilizing and adding the prednisolone acetate to the steroid before placing both into a pressurized ballmill container.

The following formulations are representative of compositions used for the treatment of angiogenesis dependent diseases.

### Example 5

### FORMULATION FOR ORAL ADMINISTRATION

### Tablet:

10-1000 mg of angiostatic steroid with inactive ingredients such as starch, lactose and magnesium stearate can be formulated according to procedures known to those skilled in the art of tablet formulation.

### Example 6

### FORMULATION FOR STERILE INTRAOCULAR INJECTION

| each mL contains: | |
|---|---|
| Angiostatic Steroid | 10-100 mg |
| Sodium Chloride | 7.14 mg |
| Potassium Chloride | 0.38 mg |
| Calcium chloride dihydrate | 0.154 mg |
| Magnesium chloride hexahydrate | 0.2 mg |
| Dried sodium phosphate | 0.42 mg |
| Sodium bicarbonate | 2.1 mg |
| Dextrose | 0.92 mg |
| Hydrochloric acid or sodium hydroxideto adjust pH to approximately 7.2 | |
| Water for injection | |

### Example 7

### FORMULATION FOR TOPICAL OCULAR SOLUTION

| | |
|---|---|
| 21-Nor-5α-pregnan-3α,17α-20-triol-3-phosphate | 1.0% |
| Benzalkonium chloride | 0.01% |
| HPMC | 0.5% |
| Sodium chloride | 0.8% |
| Sodium phosphate | 0.28% |
| Edetate disodium | 0.01% |
| NaOH/HCl | q.s. pH 7.2 |
| Purified Water | q.s. 100 mL |

### Example 8

### FORMULATION FOR TOPICAL OCULAR SUSPENSION

| Ingredient | Amount (wt.%) |
|---|---|
| 4,9(11)-Pregnadien-17α,21-diol-3,20-dione-21-acetate | 1.0 |
| Tyloxapol | 0.01 to 0.05 |
| HPMC | 0.5 |
| Benzalkonium chloride | 0.01 |
| Sodium chloride | 0.8 |
| Edetate Disodium | 0.01 |
| NaOH/HCl | q.s. pH 7.4 |
| Purified Water | q.s. 100 mL |

The formulation is prepared by first placing a portion of the purified water into a beaker and heating to 90°C. The hydroxypropylmethylcellulose (HPMC) is then added to the heated water and mixed by means of vigorous vortex stirring until all of the HPMC is dispersed. The resulting mixture is then allowed to cool while undergoing mixing in order to hydrate the HPMC. The resulting solution is then sterilized by means of autoclaving in a vessel having a liquid inlet and a hydrophobic, sterile air vent filter.

The sodium chloride and the edetate disodium are then added to a second portion of the purified water and dissolved. The benzalkonium chloride is then added to the solution, and the pH of the solution is adjusted to 7.4 with 0.1M NaOH/HCl. The solution is then sterilized by means of filtration.

The 4,9(11)-Pregnadien-17a,21-diol-3,20-dione-21-acetate is sterilized by either dry heat or ethylene oxide. If ethylene oxide sterilization is selected, aeration for at least 72 hours at 50°C is necessary. The sterilized 4,9(11)-Pregnadien-17a,21-dio1-3.20-dione-21-acetate is weighed aseptically and placed into a pressurized ballmill container. The tyloxapol, in sterilized aqueous solution form, is then added to the ballmill container. Sterilized glass balls are then added to the container and the contents of the container are milled aseptically at 225 rpm for 16 hours, or until all particles are in the range of approximately 5 microns.

Under aseptic conditions, the micronized drug suspension formed by means of the preceding step is then poured into the HPMC solution with mixing. The ballmill container and balls contained therein are then rinsed with a portion of the solution containing the sodium chloride, the edetate disodium and benzalkonium chloride. The rinse is then added aseptically to the HPMC solution. The final volume of the solution is then adjusted with purified water and, if necessary, the pH of the solution is adjusted to pH 7.4 with NaOH/HCl. The formulation will be given topically, in a therapeutically effective amount. In this instance, the phrase "therapeutically effective amount" means an amount which is sufficient to substantially prevent or reverse any ocular neovascularization. The dosage regimen used will depend on the nature of the neovascularization, as well as various other factors such as the patient's age, sex, weight, and medical history.

### Example 9

### FORMULATION FOR ORAL ADMINISTRATION

### Tablet:

5-100 mg 21-Nor-5β-pregnan-3α-17α-20-triol with inactive ingredients such as starch, lactose and magnesium stearate can be formulated according to procedures known to those skilled in the art of tablet formulation.

### Example 10

### Formulation for Sterile Intraocular Injection

| each mL contains: | |
|---|---|
| 4,9(11)-Pregnadien-17α,21-diol-3,20-dione | 10-100 mg |
| Sodium Chloride | 7.14 mg |
| Potassium Chloride | 0.38 mg |
| Calcium chloride dihydrate | 0.154 mg |
| Magnesium chloride hexahydrate | 0.2 mg |
| Dried sodium phosphate | 0.42 mg |
| Sodium bicarbonate | 2.1 mg |
| Dextrose | 0.92 mg |
| Hydrochloric acid or sodium hydroxide to adjust pH to approximately 7.2 | |
| Water for injection | |

### Example 11

Inhibition of angiogenesis in the rabbit corneal neovascularization model:
The corneal pocket system of BenEzra (Am. J. Ophthalmol 86:455-461, 1978) was used to induce corneal neovascularization in the rabbit. A small Elvax pellet containing 0.5µg of lipopolysaccharide (LPS) was inserted into the middle of the corneal stroma and positioned 2.5 mm from the limbus. An additional Elvax pellet with or without 50µg of angiostatic steroid was placed next to the LPS implant. The eyes were examined daily and the area of neovascularization calculated. Results after 8 days of LPS implantation are shown in Figure 1. THF - tetrahydrocortisol; A = 4,9(11)-Pregnadien-17α,21-diol-3,20-dione-21-acetate; B = 4,9(11)-Pregnadien-17α,21-diol-3,20-dione. As can be seen, A & B totally inhibited corneal neovascularization, whereas THF partially inhibited the neovascular response.

### Example 12

### Preparation of 5β-Pregnan-11β, 17α, 21-triol-20-one

### Tetrahydrocortisol-F-21-t-butyldiphenylsilyl ether (PS03842)

A solution of 4.75 g (17.3 mmol) of t-butyldiphenylchlorosilane in 5 mL of dry DMF was added dropwise to a stirred solution of 5.7 g (15.6 mmol) of tetrahydrocortisol-F (Steraloids No. P9050) and 2.3 g (19 mmol) of 4-dimethylaminopyridine (DMAP) in 30 mL of dry DMF, under N₂, at -25 to -30°C (maintained with CO₂ - MeCN). After a further 20 min at -30°C, the mixture was allowed to warm to 23°C overnight.

The mixture was partitioned between ether and water, and the organic solution was washed with brine, dried (MgSO₄), filtered and concentrated to give 10.7 g of a white foam.

This material was purified by flash column chromatography (400 g silica; 62.5 to 70% ether/hexane). The 3-siloxy isomer eluted first, followed by mixed fractions, followed by the title compound. The concentrated mixed fractions (4.0 g) were chromatographed on the same column with 35% ethyl acetate/hexane. The total yield of the 3-siloxy isomer was 0.42 g (5%), and of the title compound, 5.05 g (53.5%). Continued elution with 25% MeOH/EtOAc allowed recovery of unreacted tetrahydrocortisol-F.

### PS03842

NMR (200 MHz ¹H) (CDCl₃): δ0.63 (s, 3H, Me-18); 1.11 (s, 9H, t-Bu); 1.12 (s, 3H, Me-19); 2.57 (t, J-13, 1H, H-8); 2.6 (s, 1H, OH-17); 3.63 (sept, J=2.5, 1H, H-3); 4.15 (br s, 1H, H-11); 4.37 and 4.75 (AB, J=20, 2H, H-21); 7.4 (m, 6H) and 7.7 (m, 4H) (Ph₂).
NMR (200 MHz ¹H) (DMSO-d₆): δ0.64 (s, 3H, Me-18); 1.02 (s, 9H, t-Bu); 1.07 (s, 3H, Me-19); 2.50 (t, J=13, 1H, H-8); 3.37 (m, 1H, H-3); 3.94 (d, J=2, 1H, OH-11); 4.00 (br s, 1H, H-11); 4.42 (d, J=5, 1H, OH-3); 4.38 and 4.83 (AB, J=20, 2H, H-21); 5.11 (s, 1H, OH-17); 7.45 (m, 6H) and 7.6 (m, 4H) (Ph₂).

NMR (50.3 - MHz ¹³C) (CDCl₃): 17.4 (C-18); 19.3 (C-16); 23.7 (C-15); 26.3 (C-7); 26.6 (C-19); 26.8 (Me₃C); 27.2 (C-6); 30.9 (C-2); 31.5 (C-8); 34.1 (Me₃C); 34.8 (C-10); 35.2 (C-1); 36.2 (C-4); 39.7 (C-13); 43.5 (C-5); 44.3 (C-9); 47.4 (C-12); 52.1 (C-14); 67.8 (C-11); 68.9 (C-21); 71.7 (C-3); 89.8 (C-14); 127.8, 129.8, 132.8, 132.9, 135.7, 135.8 (diastereotopic Ph₂); 208.8 (C-20). Underlined resonances showed inversion in the APT experiment. Assignments: E. Breitmaier, W. Voelter "Carbon-13 NMR Spectroscopy," 3d ed., VCH, 1987; pp. 345-348.

IR (KBr) 3460, 2930, 2860, 1720, 1428, 1136, 1113, 1070, 1039, 703 cm⁻¹.

This compound did not show a sharp melting point but turned to a foam at 80-100°C. Numerous attempts at recrystallization failed.

### 5β-Pregnan-11β, 17α, 21-triol-20-one

A solution of PS03842 (0.91 g, 1.50 mmol) and thiocarbonyl diimidazole (1.05 g, 5.9 mmol) in 8 mL of anhydrous dioxane was refluxed under N₂ for 3.5 h. The cooled solution was partitioned between ether and water and the organic solution was washed with brine, dried (MgSO₄), filtered and concentrated. The residue was chromatographed (120 g SiO₂, 35% EtOAc/hexane) giving 0.86 g (80%) of the imidazolyl thioester.

A solution of 0.75 g (1.05 mmol) of this compound in 100 mL of anhydrous dioxane was added dropwise over 2.2 h to a rapidly stirred, refluxing solution of 1.6 mL (5.9 mmol) of Bu₃SnH in 100 mL of anhydrous dioxane under N₂. After a further 1 h at reflux, the solution was cooled, concentrated and the residue chromatographed (200 g SiO₂, 9% EtOAc/hexane) giving 0.43 g (70%) of the 3-deoxy-21-silyl ether. This material was dissolved in 20 mL of methanol; Bu₄NF·3H₂O (0.50 g, 1.6 mmol) was added, and the mixture was heated to reflux under N₂ for 4 h. The cooled solution was diluted with 2 volumes of EtOAc, concentrated to 1/4 volume, partitioned (EtOAc/H₂O), and the organic solution was washed with brine, dried (MgSO₄), filtered and concentrated. The residue (0.40 g) was chromatographed (30 g SiO₂, 40% EtOAc/hexane) to give 0.25 g (98%) of an oil.

This oil was crystallized (n-BuCl) to afford 0.14 g of the title compound as a white solid, m.p. 167-170°C.

IR (KBr): 3413 (br), 2934, 1714, 1455, 1389, 1095, 1035 cm⁻¹.

MS (CI): 351 (M +1).

NMR (200 MHz ¹H, DMSO-d₆): δ0.69 (s, 3H, Me-18); 1.14 (s, 3H, Me-19); 0.8-2.0 (m); 2.5 (t, J=13, 1H, H-8); 3.96 (d, J=2, 1H, OH-11); 4.1 (br s, 1H, H-11); 4.1 and 4.5 (AB, further split by 5 Hz, 2H, H-21); 4.6 (t, J=5, 1H, OH-21); 5.14 (s, 1H, OH-17).

Anal. Calc'd for C₂₁H₃₄O₄: C, 71.96; H, 9.78.
Found: C, 71.69; H, 9.66.

### Example 13

### Preparation of 21-Methyl-5β-pregnan-3α, 11β, 17α, 21-tetrol-20-one 21-methyl ether

Sodium hydride (60% oil dispersion, 0.10 g, 2.5 mmol) was added to a stirred solution of tetrahydrocortisol-F (0.73 g, 2.0 mmol) and CH₃I (0.60 mL, 9.6 mmol) in 8 mL of anhydrous DMF under N₂. Hydrogen was evolved, and the temperature rose to 35°C. After 1 h, the mixture was diluted with EtOAc, extracted with water (until neutral) and brine, dried (MgSO₄), filtered and concentrated. The residue was chromatographed (70 g SiO₂, 80% EtOAc/hexane) to give 0.17 g of a white solid, MS (CI) = 395 (M +1). This material was recrystallized (EtOAc-n-BuCl) to afford 0.12 g (16%) of the title compound as a feathery white solid, m.p. 208-213 °C.

IR (KBr): 3530, 3452, 2939, 2868, 1696 (s, CO), 1456, 1366, 1049 cm⁻¹.

NMR (200 MHz ¹ H, DMSO-d₆): δ0.74 (s, 3H, Me-18); 1.09 (s, 3H, Me-19); 1.14 (d, J=6.6, 3H, C-21 Me); 0.8-2.0 (m); 2.47 (t, J=13, 1H, H-8); 3.18 (s, 3H, OMe); 3.35 (m, 1H, H-3); 4.00 (d, J=2, 1H, OH-11); 4.07 (br s, 1H, H-11); 4.37 (q, J=6.6, 1H, H-21); 4.43 (d, J=5, 1H, OH-3); 5.16 (s, 1H, OH-17).

Anal. Calc'd for C₂₃H₃₈O₅: C, 70.01; H, 9.71.
Found: C, 70.06; H, 9.76.

### Example 14

### Preparation of 3β-Azido-5β-pregnan-11β, 17α,21-triol-20-one-21-acetate

A solution of triphenylphosphine (2.6 g, 10 mmol) in 10 mL of toluene was carefully added to a stirred solution of PS03842 (see Example 4) (1.75 g, 2.90 mmol), diphenylphosphoryl azide (2.2 mL, 10.2 mmol) and diethyl azodicarboxylate (1.55 mL, 10 mmol) under N₂, keeping the internal temperature below 35°C (exothermic). The solution was stirred for 1.2 h, then diluted with ether, washed with water and brine, dried (MgSO₄), filtered and concentrated and the residue (9.5 g, oil) chromatographed 175 g SiO₂, 15% EtOAc/hexane) giving 1.83 g of a viscous oil.

A solution of 1.73 g of this material and 1.75 g (5.5 mmol) of Bu₄NF·3H₂O in 20 mL of methanol was refluxed under N₂ for 2.5 h. The crude product (1.94 g) was isolated with ethyl acetate and chromatographed (100 g SiO₂, 50% EtOAc/hexane) giving 0.60 g (56%) of a white semisolid. Trituration (4:1 hexane-ether) gave 0.57 g (53%) of a solid.

A stirred solution of 0.40 g of this material in 3 mL of dry pyridine was treated with 0.3 mL of acetic anhydride and stirred overnight at 23°C under N₂. The mixture was quenched with 1 mL of methanol, stirred for 15 min, diluted with ether, washed with 1 M aqueous HCl, water (until neutral), brine, dried (MgSO4), filtered and concentrated. The residue (0.41 g, oil) was chromatographed (35 g SiO₂, 33% EtOAc/hexane) to afford 0.33 g (76%) of the title compound as a white foam, m.p. 80-90°C (dec).

IR (KBr): 3505, 2927, 2866, 2103 (vs), 1721 (sh 1730), 1268, 1235 cm⁻¹.

NMR (200 MHz ¹H, CDCl₃): δ0.92 (s, 3H, Me-18); 1.21 (s, 3H, Me-19); 1.0-2.1 (m); 2.17 (s, 3H, Ac); 2.25 (s 1H, OH-17); 2.74 (m, 1H, H-8); 3.97 (br s, 1H, H-3); 4.31 (br s, 1H, H-11); 4.94 (AB, J=17, Δν=60, 2H, H-21).

Anal. Calc'd for C₂₃H₃₅N₃O₅: C, 63.72; H, 8.14; N, 9.69.
Found: C, 63.39; H, 8.18; N, 9.45.

### Example 15

### Preparation of 3β-Acetamido-5β-pregnan-11β, 17α-21-triol-20-one-21-acetate

A solution of 3β-azido-5β-pregnan-11β,17α,21-triol-20-one-21-acetate (0.15 g, 0.35 mmol) in 8 mL of absolute ethanol containing 0.03 g of 10% Pd on C was stirred under H₂ ( 1 atm) at 23°C for 2 h. The mixture was filtered and concentrated, the residue dissolved in EtOAc, the basic material extracted into 1 M aqueous HCl, liberated (Na₂CO₃), extracted (EtOAc) and the organic extract washed with water (until neutral) and brine, dried (MgSO₄), filtered and concentrated to provide 58 mg of a solid.

This material was acetylated (1.0 mL of dry pyridine, 0.20 mL of Ac₂O, 23°C, N₂, overnight), followed by workup (as described for the steroid of Example 14 [last step]) affording a crude product that was chromatographed (25 g SiO₂, EtOAc). This product was triturated with ether to afford 51 mg (33%) of product as a white solid, m.p. 179-181°C.

MS (CI, isobutane): (M +1) = 450 (M⁺), 432, 391, 371, 348.

IR (KBr): 3398 (br), 2932, 2865, 1720 (sh. 1740), 1652, 1538, 1375, 1265, 1236 cm⁻¹.

NMR (200 MHz ¹H, CDCl₃): δ0.89, 1.22, 1.99, 2.17 (all s, 3H); 1.0-2.2 (m); 2.7 (t, J=13, 1H, H-8); 3.03 (s, 1H, OH-17); 4.2 (br s, 1H, H-11); 4.3 (br s, 1H, H-3); 4.96 (AB, J=17.5, Δν=42, 2H, H-21); 5.8 (d, J=10, 1H, NH).

Preferred embodiments of the present invention are the following embodiments denoted as Emb-1 to Emb-49:
Emb-1. A compound of the following formula: 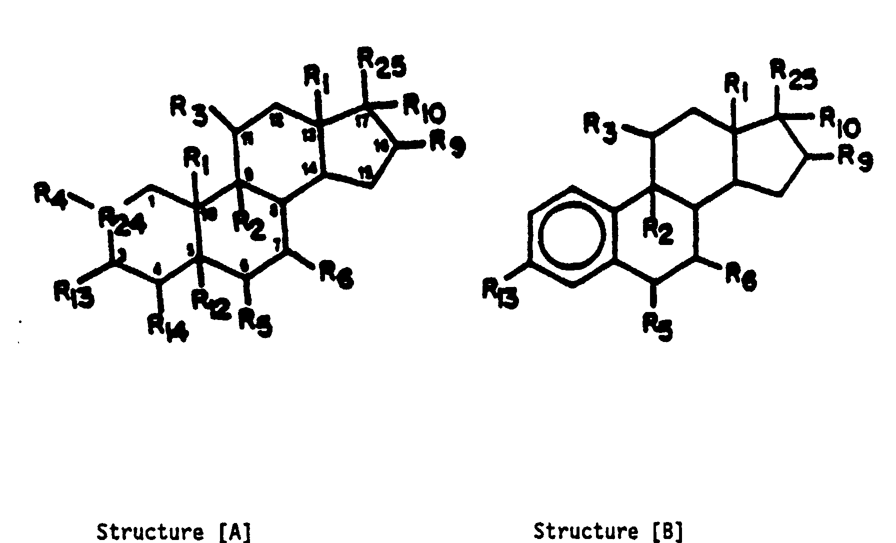 wherein R₁ is H, β-CH₃ or β-C₂H₅;
   R₂ is F, C₉-C₁₁ double bond, C₉-C₁₁ epoxy, H or Cl;
   R₃ is H, OR₂₆, OC(=O)R₂₇, halogen, C₉-C₁₁ double bond, C₉-C₁₁ epoxy, =O, -OH, -O-alkyl(C₁-C₁₂), -OC(=O)alkyl(C₁-C₁₂), -OC(=O)ARYL, -OC(=O)N(R)₂ or -OC(=O)OR₇, wherein ARYL is furyl, thienyl, pyrrolyl, or pyridyl and each of said moieties is optionally substituted with one or two (C₁-C₄)alkyl groups, or ARYL is -(CH₂)_{f}-phenyl wherein f is 0 to 2 and the phenyl ring is optionally substituted with 1 to 3 groups selected from chlorine, fluorine, bromine, alkyl(C₁-C₃), alkoxy(C₁-C₃), thioalkoxy-(C₁-C₃), Cl₃C-, F₃C-, -NH₂ and -NHCOCH₃ and R is hydrogen, alkyl (C₁-C₄), or phenyl and each R can be the same or different, and R₇ is ARYL as herein defined, or alkyl(C₁-C₁₂);
   R₄ is H, CH₃, Cl or F;
   R₅ is H, OH, F, Cl, Br, CH₃, phenyl, vinyl or allyl;
   R₆ is H or CH₃;
   R₉ is CH₂CH₂OR₂₆, CH₂CH₂OC(=O)R₂₇, H, OH, CH₃, F, =CH₂, CH₂C(=O)OR₂₈, OR₂₆, O(C=O)R₂₇ or O(C=O)CH₂(C=O)OR₂₆
   R₁₀ is -C≡CH, -CH=CH₂, halogen, CN, N₃, OR₂₆, OC(=O)R₂₇, H, OH, CH₃ or R₁₀ forms a second bond between positions C-16 and C-17;
   R₁₂ is H or forms a double bond with R₁ or R₁₄;
   R₁₃ is halogen, OR₂₆, OC(=O)R₂₇, NH₂, NHR₂₆, NHC(=O)R₂₇, N(R₂₆)₂, NC(=O)R₂₇, N₃,
   H, -OH =O, -O-P(=O)(OH)₂, or -O-C(=O)-(CH₂)ₜCOOH where t is an integer from 2 to 6;
   R₁₄ is H or forms a double bond with R₁₂;
   R₁₅ is H, =O or -OH;
   and R₂₃ with R₁₀ forms a cyclic phosphate;
   wherein R₉ and R₁₅ have the meaning defined above;
   or wherein R₂₃ is -OH, O-C(=O)-R₁₁, -OP(O)-(OH)₂, or -O-C(=O)-(CH₂)ₜCOOH
   wherein t is an integer from 2 to 6; and R₁₁ is -Y-(CH₂)ₙ-X-(CH₂)ₘ-SO₃H, -Y'-(CH₂)ₚ-X'-(CH₂)_{q}-NR₁₆R₁₇ or -Z(CH₂)ᵣQ,
   wherein Y is a bond or -O-; Y' is a bond, -O-, or -S-; each of X and X' is a bond, -CON(R₁₈)-, -N(R₁₈)CO-, -O-, -S-, -S(O)-, or -S(O₂)-; R₁₈ is hydrogen or alkyl (C₁-C₄); each of R₁₆ and R₁₇ is a lower alkyl group of from 1 to 4 carbon atoms optionally substituted with one hydroxyl or R₁₆ and R₁₇ taken together with the nitrogen atom to which each is attached forms a monocyclic heterocycle selected from pyrrolidino, piperidino, morpholino, thiomorpholino, piperazino or N(lower)alkyl-piperazino wherein alkyl has from 1 to 4 carbon atoms; n is an integer of from 4 to 9; m is an integer of from 1 to 5; p is an integer of from 2 to 9; q is an integer of from 1 to 5;
   Z is a bond or -O-; r is an integer of from 2 to 9; and Q is one of the following:
   (1) -R₁₉-CH₂COOH wherein R₁₉ is -S-, -S(O)-, -S(O)₂-, -SO₂N(R₂₀)-, or N(R₂₀)SO₂-; and R₂₀ is hydrogen or lower alkyl-(C₁-C₄); with the proviso that the total number of carbon atoms in R₂₀ and (CH₂)ᵣ is not greater than 10; or
   (2) -CO-COOH; or
   (3) CON(R₂₁)CH(R₂₂)COOH wherein R₂₁ is H and R₂₂ is H, CH₃, -CH₂COOH, -CH₂CH₂COOH, -CH₂OH, -CH₂SH, -CH₂CH₂SCH₃, or
      -CH₂Ph-OH wherein Ph-OH is p-hydroxyphenyl;
      or R₂₁ is CH₃ and R₂₂ is H;
      or R₂₁ and R₂₂ taken together are -CH₂CH₂CH₂-;
      or -N(R₂₁)CH(R₂₂)COOH taken together is -NHCH₂CONHCH₂COOH; and pharmaceutically acceptable salts thereof;
   with the proviso that except for the compound wherein R₁ is β-CH₃, R₂ and R₃ taken together form a double bond between positions 9 and 11, R₄ and R₆ are hydrogen, R₁₂ and R₁₄ taken together form a double bond between positions 4 and 5, R₅ is α-F, R₉ is β-CH₃, R₁₀ is α-OH, R₁₃ and R₁₅ are =O and R₂₃ is -OP(O)-(OH)₂, R₁₃ is =O only when R₂₃ with R₁₀ forms the above described cyclic phosphate.
   - R₂₄ =: C, C₁-C₂ double bond, O;
   - R₂₅ =: C(R₁₅)CH₂-R₂₃, OH, OR₂₆, OC(=O)R₂₇, R₂₆, COOH, C(=O)OR₂₆, CHOHCH₂OH, CHOHCH₂OR₂₆, CHOHCH₂OC(=O)R₂₇, CH₂CH₂OH, CH₂CH₂OR₂₆, CH₂CH₂OC(=O)R₂₇, CH₂CN, CH₂N₃, CH₂NH₂, CH₂NHR₂₆, CH₂N(R₂₆)₂, CH₂OH, CH₂OR₂₆, CH₂O(C=O)R₂₇, CH₂O(P=O) (OH)₂, CH₂O(P=O) (OR₂₆)₂, CH₂SH, CH₂S-R₂₆, CH₂SC(=O)R₂₇, CH₂NC(=O)R₂₇, C(=O)CHR₂₈OH, C(=O)CHR₂₈OR₂₆, C(=O)CHR₂₈OC(=O)R₂₇ or R₁₀ and R₂₅ taken together may be =C(R₂₈)₂, that is, an optionally alkyl substituted methylene group;
   wherein R₂₆ = C₁-C₆ (alkyl, branched alkyl, cycloalkyl, haloalkyl, aralkyl, aryl); R₂₇ = R₂₆ + OR₂₆; R₂₈ = H, C1-C6 (alkyl, branched alkyl, cycloalkyl); excepted from the compounds of Structure [A] are the compounds wherein R₁ is β-CH₃ or β-C₂H₅;
   R₂ is H or Cl;
   R₃ is H, =O, -OH, -O-alkyl(C₁-C₁₂), -OC(=O)alkyl(C₁-C₁₂), -OC(=O)ARYL, -OC(=O)N(R)₂ or α-OC(=O)OR₇, wherein ARYL is furyl, thienyl, pyrrolyl, or pyridyl and each of said moieties is optionally substituted with one or two (C₁-C₄)alkyl groups, or ARYL is -(CH₂)_{f}-phenyl wherein f is 0 to 2 and the phenyl ring is optionally substituted with 1 to 3 groups selected from chlorine, fluorine, bromine, alkyl(C₁-C₃), alkoxy(C₁-C₃), thioalkoxy-(C₁-C₃), Cl₃C-, F₃C-, -NH₂ and -NHCOCH₃ and R is hydrogen, alkyl (C₁-C₄), or phenyl and each R can be the same or different, and R₇ is ARYL as herein defined, or alkyl(C₁-C₁₂);
   or
   wherein R₂ and R₃ taken together are oxygen (-O-) bridging positions C-9 and C-11; or
   wherein R₂ and R₃ taken together form a double bond between positions C-9 and C-11;
   or R₂ is α-F and R₃ is β-OH;
   or R₂ is α-Cl and R₃ is β-Cl;
   and R₄ is H, CH₃, Cl or F;
   R₅ is H, OH, F, Cl, Br, CH₃, phenyl, vinyl or allyl;
   R₆ is H or CH₃;
   R₉ is H, OH, CH₃, F or =CH₂;
   R₁₀ is H, OH, CH₃ or R₁₀ forms a second bond between positions C-16 and C-17;
   R₁₂ is -H or forms a double bond with R₁₄;
   R₁₃ is H, -OH, =O, -O-P(O)(OH)₂, or -O-C(=O)-(CH₂)ₜCOOH where t is an integer from 2 to 6;
   R₁₄ is H or forms a double bond with R₁₂;
   R₁₅ is =O or -OH;
   and R₂₃ with R₁₀ forms a cyclic phosphate;
   wherein R₉ and R₁₅ have the meaning defined above;
   or wherein R₂₃ is -OH, O-C(=O)-R₁₁, -OP(O)-(OH)₂, or -O-C(=O)-(CH₂)ₜCOOH wherein t is an integer from 2 to 6; and R₁₁ is -Y-(CH₂)ₙ-X-(CH₂)ₘ-SO₃H, -Y'-(CH₂)ₚ-X'-(CH₂)_{q}-NR₁₆R₁₇ or -Z(CH₂)ᵣQ, wherein Y is a bond or -O-; Y' is a bond, -O-, or -S-; each of X and X' is a bond, -CON(R₁₈)-, -N(R₁₈)CO-, -O-, -S-, -S(O)-, or -S(O₂)-; R₁₈ is hydrogen or alkyl (C₁-C₄); each of R₁₆ and R₁₇ is a lower alkyl group of from 1 to 4 carbon atoms optionally substituted with one hydroxyl or R₁₆ and R₁₇ taken together with the nitrogen atom to which each is attached forms a monocyclic heterocycle selected from pyrrolidino, piperidino, morpholino, thiomorpholino, piperazino or N(lower)alkyl-piperazino wherein alkyl has from 1 to 4 carbon atoms; n is an integer of from 4 to 9; m is an integer of from 1 to 5; p is an integer of from 2 to 9; q is an integer of from 1 to 5;
   Z is a bond or -O-; r is an integer of from 2 to 9; and Q is one of the following:
   (1) -R₁₉-CH₂COOH wherein R₁₉ is -S-, -S(O)-, -S(O)₂-, -SO₂N(R₂₀)-, or N(R₂₀)SO₂-; and R₂₀ is hydrogen or lower alkyl-(C₁-C₄); with the proviso that the total number of carbon atoms in R₂₀ and (CH₂)ᵣ is not greater than 10; or
   (2) -CO-COOH; or
   (3) CON(R₂₁)CH(R₂₂)COOH wherein R₂₁ is H and R₂₂ is H, CH₃, -CH₂COOH, -CH₂CH₂COOH, -CH₂OH, -CH₂SH, -CH₂CH₂SCH₃, or
      -CH₂Ph-OH wherein Ph-OH is p-hydroxyphenyl;
      or R₂₁ is CH₃ and R₂₂ is H;
      or R₂₁ and R₂₂ taken together are -CH₂CH₂CH₂-;
      or -N(R₂₁)CH(R₂₂)COOH taken together is -NHCH₂CONHCH₂COOH; and
      pharmaceutically acceptable salts thereof;
   with the proviso that except for the compound wherein R₁ is β-CH₃, R₂ and R₃ taken together form a double bond between positions 9 and 11, R₄ and R₆ are hydrogen, R₁₂ and R₁₄ taken together form a double bond between positions 4 and 5, R₅ is α-F, R₉ is β-CH₃, R₁₀ is α-OH, R₁₃ and R₁₅ are =O and R₂₃ is -OP(O)-(OH)₂, R₁₃ is =O only when R₂₃ with R₁₀ forms the above described cyclic phosphate;
   also excepted from the compounds of Structure [A] are the compound 3,11β, 17α, 21-tetrahydroxy-5 -pregnane-20-one (the 3-α, 5-β; 3-α, 5-α; 3-β, 5-α; and 3-β, 5-β isomers of tetrahydrocortisol) wherein R₁₅ is =O, R₁₀ is α-OH, R₁ is β-CH₃, R₃ is β-OH, R₂ is H, R₄ is H, R₁₃ is α- or β-OH, R₁₄ is H, R₁₂ is α- or β-H, R₅ is H, R₆ is H, R₉ is H, R₂₄ is C, and R₂₃ is OH; and methyltestosterone, wherein R₁ is β-CH₃, R₂ is H, R₃ is H, R₄ is H, R₅ is H, R₆ is H, R₉ is H, R₁₀ is α-CH₃, R₁₂ and R₁₄ form a C₄-C₅ double bond, R₁₃ is =O, R₂₄ is C and R₂₅ is β-OH; dihydrotestosterone, wherein R₁ is β-CH₃, R₂R₃R₄R₅R₆R₉R₁₀R₁₄ are H, R₁₂ is α-H, R₁₃ is =O, R₂₄ is C, and R₂₅ is β-OH; dromostanolone propionate, wherein R₁ is β-CH₃,
   R₂R₃R₄R₅R₆R₉R₁₀R₁₄ are H, R₁₂ is α-H, R₁₃ is =O, R₂₄ is C and R₂₅ is β-OC(=O)CH₂CH₃; methandrostenelone, wherein R₁ is β-CH₃, R₂R₃R₄R₅R₆R₉ are H, R₁₀ is α-CH₃, R₁₂ and R₁₄ form a C₄C₅ double bond, R₁₃ is =O, R₂₄ is C₁C₂ double bond, and R₂₅ is β-OH; testosterone, wherein R₁ is β-CH₃, R₂R₃R₄R₅R₆R₉R₁₀ are H, R₁₂ and R₁₄ form a C₄C₅ double bond, R₁₃ is =O, R₂₄ is C, and R₂₅ is β-OH; norethandrolone, wherein R₁ is CH₃(C₁₃) and H(C₁₀), R₂R₃R₄R₅R₆ R₉ are H, R₁₀ is α-CH₂CH₃, R₁₂ and R₁₄ form a C₄-C₅ double bond, R₁₃ is =O, R₂₄ is C, and R₂₅ is β-OH; bolasterone, wherein R₁ is β-CH₃, R₂R₃R₄R₅R₉ are H, R₆ is α-CH₃, R₁₀ is α-CH₃, R₁₃ is =O, R₁₂ and R₁₄ form a C₄C₅ double bond, C₂₄ is C and R₂₅ is β-OH; and oxandrolone, wherein R₁ is β-CH₃, R₂R₃R₅R₆R₉R₁₄ are H, R₁₀ is αCH₃, R₁₂ is α-H, R₁₃ is =O, R₂₄ is O, and R₂₅ is
   β-OH.
Emb-2. The compound of Emb- 1 selected from the group consisting of: 21-Nor-5β-pregnan-3α,17α,20-triol; 21-Nor-5β-pregn-17(20)en-3α,16-diol-3-acetate-16-(O-methyl)malonate;21-Nor-5β-pregnan-3α,17α,20-triol-3-acetate; 21-Nor-5α-pregnan-3α,17α,20-triol-3-phosphate; 21-Nor-5β-pregn-17(20)en-3α,16-diol; 21-Nor-5β-pregnan-3α,17β,20-triol; 20-Acetamide-21-nor-5β-pregnan-3α,17α-diol-3-acetate;3β-Acetamido-5β-pregnan-11β,17α,21-triol-20-one-21-acetate; 21-Nor-5α-pregnan-3α,17β,20-triol; 21α-Methyl-5β-pregnan-3α,11β, 17α, 21-tetrol-20-one-21-methyl ether; 20-Azido-21-nor-5β-pregnan-3α,17α-diol; 20(Carbethoxymethyl)thio-21-nor-5β-pregnan-3α,17α-diol; 20-(4-Fluorophenyl)thio-21-nor-5β-pregnan-3α,17α-diol; 16α-(2-Hydroxyethyl)-17β-methyl-5β-androstan-3α,17α-diol;20-Cyano-21-nor-5β-pregnan-3α,17α-diol; 17α-Methyl-5β-androstan-3α,17β-diol; 21-Nor-5β-pregn-17(20)en-3α-OL; 21-Nor-5β-pregn-17(20)en-3α-ol-3-acetate; 21-Nor-5β-pregn-17(20)-en-3α-ol-16-acetic acid-3-acetate; 3β-Azido-5β-pregnan-11β, 17α, 21-triol-20-one-21-acetate; and 5β-Pregnan-11β,17α,21-triol-20-one.
Emb-3. The compound of Emb- 2 which is 21-Nor-5β-pregnan-3α,17α,20-triol and 21-Nor-5β-pregn-17(20)-en-3α,16-diol-3-acetate-16-(O-methyl) malonate.
Emb-4. A method for controlling ocular hypertension associated with primary open angle glaucoma which comprises administering a pharmaceutically effective amount of a compound of Emb-1.
Emb-5. A method for controlling ocular hypertension associated with primary open angle glaucoma, which comprises: administering a pharmaceutically effective amount of a compound selected from the group consisting of: 21-Nor-5β-pregnan-3α,17α,20-triol-3-acetate; 21-Nor-5α-pregnan-3α,17α,20-triol-3-phosphate; 21-Nor-5β-pregn-17(20)en-3α,16-diol; 21-Nor-5β-pregnan-3α,17β,20-triol; 20-Acetamide-21-nor-5β-pregnan-3α,17α-diol-3-acetate; 3β Acetamido-5β-pregnan-11β,17α,21-triol-20-one-21-acetate; 21-Nor-5α-pregnan-3α,17β,20-triol; 21α-Methyl-5β-pregnan-3α,11β, 17α, 21-tetrol-20-one-21-methyl ether; 20-Azido-21-nor-5β-pregnan-3α,17α-diol; 20(Carbethoxymethyl)thio-21-nor-5β-pregnan-3α,17α-diol; 20-(4-Fluorophenyl)thio-21-nor-5β-pregnan-3α,17α-diol; 16α-(2-Hydroxyethyl)-17β-methyl-5β-androstan-3α,17α-diol; 20-Cyano-21-nor-5β-pregnan-3α,17α-diol; 17α-Methyl-5β-androstan-3α,17β-diol;21-Nor-5β-pregn-17(20)en-3α-OL;21-Nor-5β-pregn-17(20)en-3α-ol-3-acetate; 21-Nor-5β-pregn-17(20)-en-3α-ol-16-acetic acid 3-acetate; 3β-Azido-5β-pregnan-11β, 17α, 21-triol-20-one-21-acetate; and5β-Pregnan-11β,17α,21-triol-20-one;4,9(11)-Pregnedien-17α,21-diol-3,20-dione; 4,9(11)-Pregnedien-17α,21-diol-3,20-dione-21-acetate; 4-Androsten-3-one-17β-carboxylic acid; 17α-Ethynyl-5(10)-estren-17β-ol-3-one; 17α-Ethynyl-1,3,5(10)-estratrien-3,17β-diol; 11-Epicortisol; 17α-Hydroxyprogesterone; and Tetrahydrocortexolone.
Emb-6. The method of Emb- 5 wherein the compound is 4, 9(11)-Pregnadien-17α,21-diol-3,20-dione-21-acetate.
Emb-7. A composition for controlling ocular hypertension associated with primary open angle glaucoma comprising a pharmaceutically effective amount of a compound of Emb- 1.
Emb-8. A composition for controlling ocular hypertension comprising a pharmaceutically effective amount of a compound selected from the group consisting of: 21-Nor-5β-pregnan-3α,17α,20-triol-3-acetate; 21-Nor-5α-pregnan-3α,17α,20-triol-3-phosphate; 21-Nor-5β-pregn-17(20)en-3α,16-diol; 21-Nor-5β-pregnan-3α,17β,20-triol; 20-Acetamide-21-nor-5β-pregnan-3α,17α-diol-3-acetate; 3β Acetamido-5β-pregnan-11β,17α,21-triol-20-one-21-acetate; 21-Nor-5α-pregnan-3α,17β,20-triol; 21α-Methyl-5β-pregnan-3α,11β, 17α, 21-tetrol-20-one-21-methyl ether; 20-Azido-21-nor-5β-pregnan-3α,17α-diol; 20(Carbethoxymethyl)thio-21-nor-5β-pregnan-3α,17α-diol; 20-(4-Fluorophenyl)thio-21-nor-5β-pregnan-3α,17α-diol; 16α-(2-Hydroxyethyl)-17β-methyl-5β-androstan-3α,17α-diol; 20-Cyano-21-nor-5β-pregnan-3α,17α-diol; 17α-Methyl-5β-androstan-3α,17β-diol;21-Nor-5β-pregn-17(20)en-3α-OL;21-Nor-5β-pregn-17(20)en-3α-ol-3-acetate; 21-Nor-5β-pregn-17(20)-en-3α-ol-16-acetic acid 3-acetate; 3β-Azido-5β-pregnan-11β, 17α, 21-triol-20-one-21-acetate; and5β-Pregnan-11β,17α,21-triol-20-one;4,9(11)-Pregnedien-17α,21-diol-3,20-dione; 4,9(11)-Pregnedien-17α,21-diol-3,20-dione-21-acetate; 4-Androsten-3-one-17β-carboxylicacid;17α-Ethynyl-5(10)-estren-17β-ol-3-one; 17α-Ethynyl-1,3,5(10)-estratrien-3,17β-diol; 11-Epicortisol; 17α-Hydroxyprogesterone; and Tetrahydrocortexolone.
Emb-9. The composition of Emb-7 wherein the compound is present at a concentration between 0.005 and 5.0 weight percent.
Emb-10. The composition of Emb-8 wherein the compound is 4, 9(11)-Pregnadien-17α,21-diol-3,20-dione-21-acetate.
Emb-11. The composition of Emb-8 wherein the compound is present at a concentration of between 0.005 and 5.0 weight percent.
Emb-12. A composition for preventing and treating neovascularization comprising a therapeutically effective amount of an angiostatic steroid of the following formula: 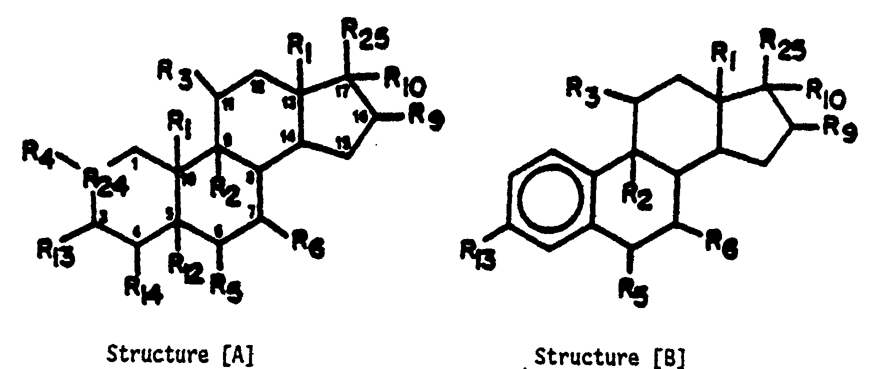 wherein R₁ is H, β-CH₃ or β-C₂H₅;
   R₂ is F, C₉-C₁₁ double bond, C₉-C₁₁ epoxy, H or -Cl;
   R₃ is H, OR₂₆, OC(=O)R₂₇, halogen, C₉-C₁₁ double bond, C₉-C₁₁ epoxy, =O, -OH, -O-alkyl(C₁-C₁₂), -OC(=O)alkyl(C₁-C₁₂), -OC(=O)ARYL, -OC(=O)N(R)₂ or -OC(=O)OR₇, wherein ARYL is furyl, thienyl, pyrrolyl, or pyridyl and each of said moieties is optionally substituted with one or two (C₁-C₄)alkyl groups, or ARYL is -(CH₂)_{f}-phenyl wherein f is 0 to 2 and the phenyl ring is optionally substituted with 1 to 3 groups selected from chlorine, fluorine, bromine, alkyl(C₁-C₃), alkoxy(C₁-C₃), thioalkoxy-(C₁-C₃), Cl₃C-, F₃C-, -NH₂ and -NHCOCH₃ and R is hydrogen, alkyl (C₁-C₄), or phenyl and each R can be the same or different, and R₇ is ARYL as herein defined, or alkyl(C₁-C₁₂);
   R₄ is H, CH₃, Cl or F;
   R₅ is H, OH, F, Cl, Br, CH₃, phenyl, vinyl or allyl;
   R₆ is H or CH₃;
   R₉ is CH₂CH₂OR₂₆, CH₂CH₂OC(=O)R₂₇, H, OH, CH₃, F, =CH₂, or CH₂C(=O)OR₂₈, OR₂₆, O(C=O)R₂₇, or O(C=O)CH₂(C=O)OR₂₆;
   R₁₀ is -C≡CH, -CH=CH₂, halogen, CN, N₃, OR₂₆, OC(=O)R₂₇, H, OH, CH₃ or R₁₀ forms a second bond between positions C-16 and C-17;
   R₁₂ is H or forms a double bond with R₁ or R₁₄;
   R₁₃ is halogen, OR₂₆, OC(=O)R₂₇, NH₂, NHR₂₆, NHC(=O)R₂₇, N(R₂₆)₂, NC(=O)R₂₇, N₃, H, -OH, =O, -O-P(=O)(OH)₂, or -O-C(=O)-(CH₂)ₜCOOH where t is an integer from 2 to 6;
   R₁₄ is H or forms a double bond with R₁₂;
   R₁₅ is H, =O or -OH;
   and R₂₃ with R₁₀ forms a cyclic phosphate;
   wherein R₉ and R₁₅ have the meaning defined above;
   or wherein R₂₃ is -OH, O-C(=O)-R₁₁, -OP(O)-(OH)₂, or -O-C(=O)-(CH₂)ₜCOOH
   wherein t is an integer from 2 to 6; and R₁₁ is -Y-(CH₂)ₙ-X-(CH₂)ₘ-SO₃H, -Y'-(CH₂)ₚ-X'-(CH₂)_{q}-NR₁₆R₁₇ or -Z(CH₂)ᵣQ,
   wherein Y is a bond or -O-; Y' is a bond, -O-, or -S-; each of X and X' is a bond, -CON(R₁₈)-, -N(R₁₈)CO-, -O-, -S-, -S(O)-, or -S(O₂)-; R₁₈ is hydrogen or alkyl (C₁-C₄); each of R₁₆ and R₁₇ is a lower alkyl group of from 1 to 4 carbon atoms optionally substituted with one hydroxyl or R₁₆ and R₁₇ taken together with the nitrogen atom to which each is attached forms a monocyclic heterocycle selected from pyrrolidino, piperidino, morpholino, thiomorpholino, piperazino or N(lower)alkyl-piperazino wherein alkyl has from 1 to 4 carbon atoms; n is an integer of from 4 to 9; m is an integer of from 1 to 5; p is an integer of from 2 to 9; q is an integer of from 1 to 5;
   Z is a bond or -O-; r is an integer of from 2 to 9; and Q is one of the following:
   (1) -R₁₉-CH₂COOH wherein R₁₉ is -S-, -S(O)-, -S(O)₂-, -SO₂N(R₂₀)-, or N(R₂₀)SO₂-; and R₂₀ is hydrogen or lower alkyl-(C₁-C₄); with the proviso that the total number of carbon atoms in R₂₀ and (CH₂)ᵣ is not greater than 10; or
   (2) -CO-COOH; or
   (3) CON(R₂₁)CH(R₂₂)COOH wherein R₂₁ is H and R₂₂ is H, CH₃, -CH₂COOH, -CH₂CH₂COOH, -CH₂OH, -CH₂SH, -CH₂CH₂SCH₃, or
      -CH₂Ph-OH wherein Ph-OH is p-hydroxyphenyl;
      or R₂₁ is CH₃ and R₂₂ is H;
      or R₂₁ and R₂₂ taken together are -CH₂CH₂CH₂-;
      or -N(R₂₁)CH(R₂₂)COOH taken together is -NHCH₂CONHCH₂COOH; and pharmaceutically acceptable salts thereof;
   with the proviso that except for the compound wherein R₁ is -CH₃, R₂ and R₃ taken together form a double bond between positions 9 and 11, R₄ and R₆ are hydrogen, R₁₂ and R₁₄ taken together form a double bond between positions 4 and 5, R₅ is -F, R₉ is -CH₃, R₁₀ is -OH, R₁₃ and R₁₅ are =O and R₂₃ is -OP(O)-(OH)₂, R₁₃ is =O only when R₂₃ with R₁₀ forms the above described cyclic phosphate;
   - R₂₄ =: C, C₁-C₂ double bond, O;
   - R₂₅ =: C(R₁₅)CH₂-R₂₃, OH, OR₂₆, OC(=O)R₂₇, R₂₆, COOH, C(=O)OR₂₆, CHOHCH₂OH, CHOHCH₂OR₂₆, CHOHCH₂OC(=O)R₂₇, CH₂CH₂OH, CH₂CH₂OR₂₆, CH₂CH₂OC(=O)R₂₇, CH₂CN, CH₂N₃, CH₂NH₂, CH₂NHR₂₆, CH₂N(R₂₆)₂, CH₂OH, CH₂OR₂₆, CH₂O(C=O)R₂₇, CH₂O(P=O)(OH)₂, CH₂O(P=O)(OR₂₆)₂, CH₂SH, CH₂S-R₂₆, CH₂SC(=O)R₂₇, CH₂NC(=O)R₂₇, C(=O)CHR₂₈OH, C(=O)CHR₂₈OH, C(=O)CHR₂₈OR₂₆, C(=O)CHR₂₈OC(=O)R₂₇ or R₁₀ and
   R₂₅ taken together may be =C(R₂₈)₂, that is, an optionally alkyl substituted methylene group;
   wherein R₂₆ = C₁-C₆ (alkyl, branched alkyl, cycloalkyl, haloalkyl, aralkyl, aryl); R₂₇ = R₂₆ + OR₂₆; R₂₈ = H, C1-C6 (alkyl, branched alkyl, cycloalkyl); excepted from the compounds of Structure [A] are the compounds wherein R₁ is β-CH₃ or β-C₂H₅;
   R₂ is H or -Cl;
   R₃ is =O, -OH, -O-alkyl(C₁-C₁₂), -OC(=O)alkyl(C₁-C₁₂), -OC(=O)ARYL, -OC(=O)N(R)₂ or α-OC(=O)OR₇, wherein ARYL is furyl, thienyl, pyrrotyl, or pyridyl and each of said moieties is optionally substituted with one or two (C₁-C₄)alkyl groups, or ARYL is -(CH₂)_{f}-phenyl wherein f is 0 to 2 and the phenyl ring is optionally substituted with 1 to 3 groups selected from chlorine, fluorine, bromine, alkyl(C₁-C₃), alkoxy(C₁-C₃), thioalkoxy-(C₁-C₃), Cl₃C-, F₃C-, -NH₂ and -NHCOCH₃ and R is hydrogen, alkyl (C₁-C₄), or phenyl and each R can be the same or different, and R₇ is ARYL as herein defined, or alkyl (C₁-C₁₂);
   or
   wherein R₂ and R₃ taken together are oxygen (-O-) bridging positions C-9 and C-11; or
   wherein R₂ and R₃ taken together form a double bond between positions C-9 and C-11;
   or R₂ is α-F and R₃ is β-OH;
   or R₂ is α-Cl and R₃ is β-Cl;
   and R₄ is H, CH₃, Cl or F;
   R₅ is H, OH, F, Cl, Br, CH₃, phenyl, vinyl or allyl;
   R₆ is H or CH₃;
   R₉ is H, OH, CH₃, F or =CH₂;
   R₁₀ is H, OH, CH₃ or R₁₀ forms a second bond between positions C-16 and C-17;
   R₁₂ is -H or forms a double bond with R₁₄;
   R₁₃ is H, -OH, =O, -O-P(O)(OH)₂, or -O-C(=O)-(CH₂)ₜCOOH where t is an integer from 2 to 6;
   R₁₄ is H or forms a double bond with R₁₂;
   R₁₅ is =O or -OH;
   and R₂₃ with R₁₀ forms a cyclic phosphate;
   wherein R₉ and R₁₅ have the meaning defined above;
   or wherein R₂₃ is -OH, O-C(=O)-R₁₁, -OP(O)-(OH)₂, or -O-C(=O)-(CH₂)ₜCOOH wherein t is an integer from 2 to 6; and R₁₁ is -Y-(CH₂)ₙ-X-(CH₂)ₘ-SO₃H, -Y'-(CH₂)ₚ-X'-(CH₂)_{q}-NR₁₆R₁₇ or -Z(CH₂)ᵣQ, wherein Y is a bond or -O-; Y' is a bond, -O-, or -S-; each of X and X' is a bond, -CON(R₁₈)-, -N(R₁₈)CO-, -O-, -S-, -S(O)-, or -S(O₂)-; R₁₈ is hydrogen or alkyl (C₁-C₄); each of R₁₆ and R₁₇ is a lower alkyl group of from 1 to 4 carbon atoms optionally substituted with one hydroxyl or R₁₆ and R₁₇ taken together with the nitrogen atom to which each is attached forms a monocyclic heterocycle selected from pyrrolidino, piperidino, morpholino, thiomorpholino, piperazino or N(lower)alkyl-piperazino wherein alkyl has from 1 to 4 carbon atoms; n is an integer of from 4 to 9; m is an integer of from 1 to 5; p is an integer of from 2 to 9; q is an integer of from 1 to 5;
   Z is a bond or -O-; r is an integer of from 2 to 9; and Q is one of the following:
   (1) -R₁₉-CH₂COOH wherein R₁₉ is -S-, -S(O)-, -S(O)₂-, -SO₂N(R₂₀)-, or N(R₂₀)SO₂-; and R₂₀ is hydrogen or lower alkyl-(C₁-C₄); with the proviso that the total number of carbon atoms in R₂₀ and (CH₂)ᵣ is not greater than 10; or
   (2) -CO-COOH; or
   (3) CON(R₂₁)CH(R₂₂)COOH wherein R₂₁ is H and R₂₂ is H, CH₃, -CH₂COOH, -CH₂CH₂COOH, -CH₂OH, -CH₂SH, -CH₂CH₂SCH₃, or -CH₂Ph-OH wherein Ph-OH is p-hydroxyphenyl;
      or R₂₁ is CH₃ and R₂₂ is H;
      or R₂₁ and R₂₂ taken together are -CH₂CH₂CH₂-;
      or -N(R₂₁)CH(R₂₂)COOH taken together is -NHCH₂CONHCH₂COOH; and pharmaceutically acceptable salts thereof;
   with the proviso that except for the compound wherein R₁ is β-CH₃, R₂ and R₃ taken together form a double bond between positions 9 and 11, R₄ and R₆ are hydrogen, R₁₂ and R₁₄ taken together form a double bond between positions 4 and 5, R₅ is α-F, R₉ is β-CH₃, R₁₀ is α-OH, R₁₃ and R₁₅ are =O and R₂₃ is -OP(O)-(OH)₂, R₁₃ is =O only when R₂₃ with R₁₀ forms the above described cyclic phosphate;
   also excepted from the compounds of Structure [A] are the compounds 3,11 β, 17α, 21-tetrahydroxy-5 -pregnane-20-one (the 3-alpha, 5-beta; 3-alpha, 5-alpha; 3-beta, 5-alpha; and 3-beta, 5-beta isomers of tetrahydrocortisol) wherein R₁₅ is =O, R₁₀ is α OH, R₁ is CH₃, R₃ is β OH, R₂ is H, R₄ is H, R₁₃ is α or β OH, R₁₄ is H, R₁₂ is α or β H, R₅ is H, R₆ is H, R₉ is H, R₂₄ is C, and
   R23 is OH.
Emb-13. A composition for preventing or treating neovascularization, comprising: a therapeutically effective amount of an angiostatic steroid selected from the group consisting of: 21-Nor-5β-pregnan-3α,17α,20-triol-3-acetate; 21-Nor-5α-pregnan-3α,17α,20-triol-3-phosphate; 21-Nor-5β-pregn-17(20)en-3α,16-diol;21-Nor-5β-pregnan-3α,17β,20-triol;20-Acetamide-21-nor-5β-pregnan-3α,17α-diol-3-acetate;3β Acetamido-5β-pregnan-11β,17α,21-triol-20-one-21-acetate;21-Nor-5α-pregnan-3α,17β,20-triol;21α-Methyl-5β-pregnan-3α,11β, 17α, 21-tetrol-20-one-21-methyl ether; 20-Azido-21-nor-5β-pregnan-3α,17α-diol; 20(Carbethoxymethyl)thio-21-nor-5β-pregnan-3α,17α-diol; 20-(4-Fluorophenyl)thio-21-nor-5β-pregnan-3α,17α-diol; 16α-(2-Hydroxyethyl)-17β-methyl-5β-androstan-3α,17α-diol; 20-Cyano-21-nor-5β-pregnan-3α,17α-diol; 17α-Methyl-5β-androstan-3α,17β-diol;21-Nor-5β-pregn-17(20)en-3α-OL;21-Nor-5β-pregn-17(20)en-3α-ol-3-acetate; 21-Nor-5β-pregn-17(20)-en-3α-ol-16-acetic acid 3-acetate; 3β-Azido-5β-pregnan-11β, 17α, 21-triol-20-one-21-acetate; and5β-Pregnan-11β,17α,21-triol-20-one;4,9(11)-Pregnedien-17α,21-diol-3,20-dione; 4,9(11)-Pregnedien-17α,21-diol-3,20-dione-21-acetate; 4-Androsten-3-one-17β-carboxylic acid; 17α-Ethynyl-5(10)-estren-17β-ol-3-one; 17α-Ethynyl-1,3,5(10)-estratrien-3,17β-diol; 11-Epicortisol; 17α-Hydroxyprogesterone; and Tetrahydrocortexolone.
Emb-14. The composition of Emb-12 wherein the angiostatic steroid concentration is 0.01 - 10.0 wt.%.
Emb-15. The composition of Emb-14 wherein the concentration is 0.1 - 5.0 wt.%.
Emb-16. A method for preventing and treating neovascularization, which comprises: administering a therapeutically effective amount of the composition of Emb-12.
Emb-17. The method for preventing and treating neovascularization which comprises: administering a therapeutically effective amount of the composition of Emb-13.
Emb-18. The method of Emb- 16 wherein the neovascularization being prevented and treated is selected from the group consisting of head trauma, spinal trauma, septic or traumatic shock, stroke, hemorrhagic shock, cancer, arthritis, arteriosclerosis, angiofibroma, arteriovenous malformations, corneal graft neovascularization, delayed wound healing, diabetic retinopathy, granulations, burns, hemangioma, hemophilic joints, hypertrophic scars, neovascular glaucoma, nonunion fractures, Osler-Weber Syndrome, psoriasis, pyogenic granuloma, retrolental fibroplasia, scleroderma, solid tumors, trachoma, vascular adhesions, and solid tumor growth.
Emb-19. A method for preventing and treating ocular neovascularization, which comprises: administering a therapeutically effective amount of the composition of Emb-12.
Emb-20. A method for preventing and treating ocular neovascularization, which comprises: administering a therapeutically effective amount of the composition of Emb-13.
Emb-21. The method of Emb- 19 wherein the angiostatic steroid concentration is 0.01 - 10 wt.%.
Emb-22. The method of Emb-19 wherein the angiostatic steroid concentration is 0.1 to 5.0 wt.%.
Emb-23. The method of Emb-19 wherein the ocular neovascularization being prevented and treated is in the front of the eye.
Emb- 24. The method of Emb-19 wherein the ocular neovascularization being treated is in the cornea.
Emb- 25. The method of Emb-19 wherein the ocular neovascularization being prevented and treated is in the back of the eye.
Emb-26. The method of Emb-17 wherein the angiostatic steroid is 4.9(11)-Pregnadien-17a,21-diol-3,20-dione-21-acetate.
Emb-27. The method of Emb-17 wherein the angiostatic steroid is 4,9(11)-Pregnadien-17a,21-diol-3,20-dione.
Emb-28. The method of Emb-17 wherein the angiostatic steroid is administered at a concentration of about 0.01 to 10.0 weight percent.
Emb-29. The method of Emb-17 wherein the angiostatic steroid is administered at a concentration of about 0.1 - 5.0 weight percent.
Emb-30. A method for preventing and treating neovascularization of the tissues in the front of the eye, which comprises: administering a pharmaceutically effective amount of a composition of Emb-13.
Emb-31. The method of Emb-30 wherein the angiostatic steroid is 4,9(11)-Pregnadien-17a,21-diol-3,20-dione-21-acetate.
Emb-32. The method of Emb-30 wherein the angiostatic steroids is 4,9(11)-Pregnadien-17a,21-diol-3,20-dione.
Emb-33. The method of Emb-30 wherein the angiostatic steroid is administered at a concentration of about 0.01 to 10.0 weight percent.
Emb-34. The method of Emb-31 wherein the concentration is about 0.1 - 5.0 weight percent.
Emb-35. The method of Emb-30 wherein the tissue being treated is the cornea.
Emb-36. A method for preventing and treating neovascularization of the tissues of the back of the eye, which comprises: administering a pharmaceutically effective amount of a composition of Emb-13.
Emb-37. The method of Emb-36 wherein the angiostatic steroid is 4,9(11)-Pregnadien-17a,21-diol-3,20-dione.
Emb-38. The method of Emb-36 wherein the angiostatic steroid is administered at a concentration of about 0.01 to 10.0 weight percent.
Emb-39. The method of Emb-38 wherein the anglostatic steroid is administered at a concentration of about 0.1 to 5.0 weight percent.
Emb-40. The method of Emb-36 wherein the tissue being treated is the retina or the subretina.
Emb-41. The method of Emb-36 wherein the tissue being treated is the macula.
Emb-42. The method of Emb-36 wherein the tissue being treated is the optic nerve head.
Emb-43. A pharmaceutical composition useful in the treatment of ophthalmic inflammation, comprising: an antiinflammatory effective amount of a glucocorticoid and an intraocular pressure controlling amount of an anglostatic steroid of the formula: 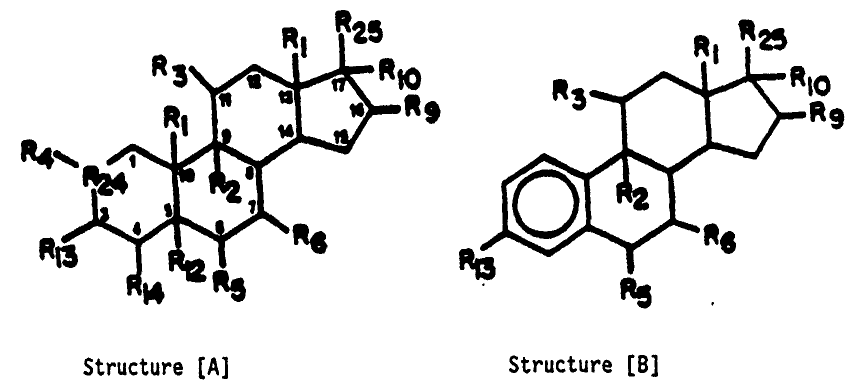 wherein R₁ is H, β-CH₃ or β-C₂H₅;
   R₂ is F, C₉-C₁₁ double bond, C₉-C₁₁ epoxy, H or Cl;
   R₃ is H, OR₂₆, OC(=O)R₂₇, halogen, C₉-C₁₁ double bond, C₉-C₁₁ epoxy, =O, -OH, -O-alkyl(C₁-C₁₂), -OC(=O)alkyl(C₁-C₁₂), -OC(=O)ARYL, -OC(=O)N(R)₂ or -OC(=O)OR₇, wherein ARYL is furyl, thienyl, pyrrolyl, or pyridyl and each of said moieties is optionally substituted with one or two (C₁-C₄)alkyl groups, or ARYL is -(CH₂)_{f}-phenyl wherein f is 0 to 2 and the phenyl ring is optionally substituted with 1 to 3 groups selected from chlorine, fluorine, bromine, alkyl(C₁-C₃), alkoxy(C₁-C₃), thioalkoxy-(C₁-C₃), Cl₃C-, F₃C-, -NH₂ and -NHCOCH₃ and R is hydrogen, alkyl (C₁-C₄), or phenyl and each R can be the same or different, and R₇ is ARYL as herein defined, or alkyl(C₁-C₁₂);
   R₄ is H, CH₃, Cl or F;
   R₅ is H, OH, F, Cl, Br, CH₃, phenyl, vinyl or allyl;
   R₆ is H or CH₃;
   R₉ is CH₂CH₂OR₂₆, CH₂CH₂OC(=O)R₂₇, H, OH, CH₃, F, =CH₂, CH₂C(=O)OR₂₈, OR₂₆, O(C=O)R₂₇ or O(C=O)CH₂(C=O)OR₂₆
   R₁₀ is -C≡CH, -CH=CH₂, halogen, CN, N₃, OR₂₆, OC(=O)R₂₇, H, OH, CH₃ or R₁₀ forms a second bond between positions C-16 and C-17;
   R₁₂ is H or forms a double bond with R₁ or R₁₄;
   R₁₃ is halogen, OR₂₆, OC(=O)R₂₇, NH₂, NHR₂₆, NHC(=O)R₂₇, N(R₂₆)₂, NC(=O)R₂₇, N₃, H, -OH, =O, -O-P(=O)(OH)₂, or -O-C(=O)-(CH₂)ₜCOOH where t is an integer from 2 to 6;
   R₁₄ is H or forms a double bond with R₁₂;
   R₁₅ is H, =O or -OH;
   and R₂₃ with R₁₀ forms a cyclic phosphate;
   wherein R₉ and R₁₅ have the meaning defined above;
   or wherein R₂₃ is -OH, O-C(=O)-R₁₁, -OP(O)-(OH)₂, or -O-C(=O)-(CH₂)ₜCOOH wherein t is an integer from 2 to 6; and R₁₁ is -Y-(CH₂)ₙ-X-(CH₂)ₘ-SO₃H, -Y'-(CH₂)ₚ-X'-(CH₂)_{q}-NR₁₆R₁₇ or -Z(CH₂)ᵣQ,
   wherein Y is a bond or -O-; Y' is a bond, -O-, or -S-; each of X and X' is a bond, -CON(R₁₈)-, -N(R₁₈)CO-, -O-, -S-, -S(O)-, or -S(O₂)-; R₁₈ is hydrogen or alkyl (C₁-C₄); each of R₁₆ and R₁₇ is a lower alkyl group of from 1 to 4 carbon atoms optionally substituted with one hydroxyl or R₁₆ and R₁₇ taken together with the nitrogen atom to which each is attached forms a monocyclic heterocycle selected from pyrrolidino, piperidino, morpholino, thiomorpholino, piperazino or N(lower)alkyl-piperazino wherein alkyl has from 1 to 4 carbon atoms; n is an integer of from 4 to 9; m is an integer of from 1 to 5; p is an integer of from 2 to 9; q is an integer of from 1 to 5;
   Z is a bond or -O-; r is an integer of from 2 to 9; and Q is one of the following:
   (1) -R₁₉-CH₂COOH wherein R₁₉ is -S-, -S(O)-, -S(O)₂-, -SO₂N(R₂₀)-, or N(R₂₀)SO₂-; and R₂₀ is hydrogen or lower alkyl-(C₁-C₄); with the proviso that the total number of carbon atoms in R₂₀ and (CH₂)ᵣ is not greater than 10; or
   (2) -CO-COOH; or
   (3) CON(R₂₁)CH(R₂₂)COOH wherein R₂₁ is H and R₂₂ is H, CH₃, -CH₂COOH, -CH₂CH₂COOH, -CH₂OH, -CH₂SH, -CH₂CH₂SCH₃, or
      -CH₂Ph-OH wherein Ph-OH is p-hydroxyphenyl;
      or R₂₁ is CH₃ and R₂₂ is H;
      or R₂₁ and R₂₂ taken together are -CH₂CH₂CH₂-;
      or -N(R₂₁)CH(R₂₂)COOH taken together is -NHCH₂CONHCH₂COOH; and pharmaceutically acceptable salts thereof;
   with the proviso that except for the compound wherein R₁ is β-CH₃, R₂ and R₃ taken together form a double bond between positions 9 and 11, R₄ and R₆ are hydrogen, R₁₂ and R₁₄ taken together form a double bond between positions 4 and 5, R₅ is α-F, R₉ is β-CH₃, R₁₀ is α-OH, R₁₃ and R₁₅ are =O and R₂₃ is -OP(O)-(OH)₂, R₁₃ is =O only when R₂₃ with R₁₀ forms the above described cyclic phosphate.
   - R₂₄ =: C, C₁-C₂ double bond, 0;
   - R₂₅ =: C(R₁₅)CH₂-R₂₃, OH, OR₂₆, OC(=O)R₂₇, R₂₆, COOH, C(=O)OR₂₆, CHOHCH₂OH, CHOHCH₂OR₂₆, CHOHCH₂OC(=O)R₂₇, CH₂CH₂OH, CH₂CH₂OR₂₆, CH₂CH₂OC(=O)R₂₇, CH₂CN, CH₂N₃, CH₂NH₂, CH₂NHR₂₆, CH₂N(R₂₆)₂, CH₂OH, CH₂OR₂₆, CH₂O(C=O)R₂₇, CH₂O(P=O) (OH)₂, CH₂O(P=O) (OR₂₆)₂, CH₂SH, CH₂S-R₂₆, CH₂SC(=O)R₂₇, CH₂NC(=O)R₂₇, C(=O)CHR₂₈OH, C(=O)CHR₂₈OR₂₆, C(=O)CHR₂₈OC(=O)R₂₇ or R₁₀ and R₂₅ taken together may be =C(R₂₈)₂, that is, an optionally alkyl substituted methylene group;
   wherein R₂₆ = C₁-C₆ (alkyl, branched alkyl, cycloalkyl, haloalkyl, aralkyl, aryl); R₂₇ = R₂₆ + OR₂₆; R₂₈ = H, C1-C6 (alkyl, branched alkyl, cycloalkyl); excepted from the compounds of Structure [A] are the compounds wherein R₁ is β-CH₃ or β-C₂H₅;
   R₂ is H or Cl;
   R₃ is H, =O, -OH, -O-alkyl(C₁-C₁₂), -OC(=O)alkyl(C₁-C₁₂), -OC(=O)ARYL, -OC(=O)N(R)₂ or α-OC(=O)OR₇, wherein ARYL is furyl, thienyl, pyrrolyl, or pyridyl and each of said moieties is optionally substituted with one or two (C₁-C₄)alkyl groups, or ARYL is -(CH₂)_{f}-phenyl wherein f is 0 to 2 and the phenyl ring is optionally substituted with 1 to 3 groups selected from chlorine, fluorine, bromine, alkyl(C₁-C₃), alkoxy(C₁-C₃), thioalkoxy-(C₁-C₃), Cl₃C- F₃C-, -NH₂ and -NHCOCH₃ and R is hydrogen, alkyl (C₁-C₄), or phenyl and each R can be the same or different, and R₇ is ARYL as herein defined, or alkyl(C₁-C₁₂);
   or
   wherein R₂ and R₃ taken together are oxygen (-O-) bridging positions C-9 and C-11; or
   wherein R₂ and R₃ taken together form a double bond between positions C-9 and C-11;
   or R₂ is α-F and R₃ is β-OH;
   or R₂ is α-Cl and R₃ is β-Cl;
   and R₄ is H, CH₃, Cl or F;
   R₅ is H, OH, F, Cl, Br, CH₃, phenyl, vinyl or allyl;
   R₆ is H or CH₃;
   R₉ is H, OH, CH₃, F or =CH₂;
   R₁₀ is H, OH, CH₃ or R₁₀ forms a second bond between positions C-16 and C-17;
   R₁₂ is -H or forms a double bond with R₁₄;
   R₁₃ is H, -OH, =O, -O-P(O)(OH)₂, or -O-C(=O)-(CH₂)ₜCOOH where t is an integer from 2 to 6;
   R₁₄ is H or forms a double bond with R₁₂;
   R₁₅ is =O or -OH;
   and R₂₃ with R₁₀ forms a cyclic phosphate;
   wherein R₉ and R₁₅ have the meaning defined above;
   or wherein R₂₃ is -OH, O-C(=O)-R₁₁, -OP(O)-(OH)₂, or -O-C(=O)-(CH₂)ₜCOOH wherein t is an integer from 2 to 6; and R₁₁ is -Y-(CH₂)ₙ-X-(CH₂)ₘ-SO₃H, -Y'-(CH₂)ₚ-X'-(CH₂)_{q}-NR₁₆R₁₇ or -Z(CH₂)ᵣQ, wherein Y is a bond or -O-; Y' is a bond, -O-, or -S-; each of X and X' is a bond,-CON(R₁₈)-, -N(R₁₈)CO-, -O-, -S-, -S(O)-, or -S(O₂)-; R₁₈ is hydrogen or alkyl (C₁-C₄); each of R₁₈ and R₁₇ is a lower alkyl group of from 1 to 4 carbon atoms optionally substituted with one hydroxyl or R₁₆ and R₁₇ taken together with the nitrogen atom to which each is attached forms a monocyclic heterocycle selected from pyrrolidino, piperidino, morpholino, thiomorpholino, piperazino or N(lower)alkyl-piperazino wherein alkyl has from 1 to 4 carbon atoms; n is an integer of from 4 to 9; m is an integer of from 1 to 5; p is an integer of from 2 to 9; q is an integer of from 1 to 5;
   Z is a bond or -O-; r is an integer of from 2 to 9; and Q is one of the following:
   (1) -R₁₉-CH₂COOH wherein R₁₉ is -S-, -S(O)-, -S(O)₂-, -SO₂N(R₂₀)-, or N(R₂₀)SO₂-; and R₂₀ is hydrogen or lower alkyl-(C₁-C₄); with the proviso that the total number of carbon atoms in R₂₀ and (CH₂)ᵣ is not greater than 10; or
   (2) -CO-COOH; or
   (3) CON(R₂₁)CH(R₂₂)COOH wherein R₂₁ is H and R₂₂ is H, CH₃, -CH₂COOH, -CH₂CH₂COOH, -CH₂OH, -CH₂SH, -CH₂CH₂SCH₃, or
      -CH₂Ph-OH wherein Ph-OH is p-hydroxyphenyl;
      or R₂₁ is CH₃ and R₂₂ is H;
      or R₂₁ and R₂₂ taken together are -CH₂CH₂CH₂-;
      or -N(R₂₁)CH(R₂₂)COOH taken together is -NHCH₂CONHCH₂COOH; and pharmaceutically acceptable salts thereof;
   with the proviso that except for the compound wherein R₁ is β-CH₃, R₂ and R₃ taken together form a double bond between positions 9 and 11, R₄ and R₆ are hydrogen, R₁₂ and R₁₄ taken together form a double bond between positions 4 and 5, R₅ is α-F, R₉ is β-CH₃, R₁₀ is α-OH, R₁₃ and R₁₅ are =O and R₂₃ is -OP(O)-(OH)₂, R₁₃ is =O only when R₂₃ with R₁₀ forms the above described cyclic phosphate.
Emb-44. The pharmaceutical composition of Emb-43, wherein the angiostatic steroid is selected from the group consisting of: 21-Nor-5β-pregnan-3α,17α,20-triol-3-acetate; 21-Nor-5α-pregnan-3α,17α,20-triol-3-phosphate; 21-Nor-5β-pregn-17(20)en-3α,16-diol; 21-Nor-5β-pregnan-3α,17β,20-triol; 20-Acetamide-21-nor-5β-pregnan-3α,17α-diol-3-acetate; 3β Acetamido-5β-pregnan-11β,17α,21-triol-20-one-21-acetate; 21-Nor-5α-pregnan-3α,17β,20-triol; 21α-Methyl-5β-pregnan-3α,11β, 17α, 21-tetrol-20-one-21-methyl ether; 20-Azido-21-nor-5β-pregnan-3α,17α-diol; 20(Carbethoxymethyl)thio-21-nor-5β-pregnan-3α,17α-diol; 20-(4-Fluorophenyl)thio-21-nor-5β-pregnan-3α,17α-diol; 16α-(2-Hydroxyethyl)-17β-methyl-5β-androstan-3α,17α-diol; 20-Cyano-21-nor-5β-pregnan-3α,17α-diol; 17α-Methyl-5β-androstan-3α,17β-diol; 21-Nor-5β-pregn-17(20)en-3α-OL; 21-Nor-5β-pregn-17(20)en-3α-ol-3-acetate; 21-Nor-5β-pregn-17(20)-en-3α-ol-16-acetic acid 3-acetate; 3β-Azido-5β-pregnan-11β, 17α, 21-triol-20-one-21-acetate; and 5β-Pregnan-11β,17α,21-triol-20-one; 4,9(11)-Pregnedien-17α,21-diol-3,20-dione; 4,9(11)-Pregnedien-17α,21-diol-3,20-dione-21-acetate; 4-Androsten-3-one-17β-carboxylic acid; 17α-Ethynyl-5(10)-estren-17β-ol-3-one; 17α-Ethynyl-1,3,5(10)-estratrien-3,17β-diol; 11-Epicortisol; 17α-Hydroxyprogesterone; Tetrahydrocortexolone; and Tetrahydrocortisol.
Emb-45. The pharmaceutical composition of Emb-44, wherein the anglostatic steroid is selected from the group consisting of: tetrahydrocortisol; 21-Nor-5β-pregnan-3α,17α,20-triol; 4,9(11)-Pregnadien-17α,21-diol-3,20-dione; 4,9(11)-Pregnadien-17α,21-diol-3,20-dione-21-acetate.
Emb-46. The pharmaceutical composition of Emb-45 wherein the angiostatic steroid is selected from the group consisting of: tetrahydrocortisol and 4,9(11)-Pregnadien-17α-21-diol-3,20-dione-21-acetate.
Emb-47. A method of treating ophthalmic Inflammation without significantly increasing the patient's intraocular pressure, which comprises: administering the composition of Emb-43.
Emb-48. A method of treating ophthalmic inflammation without significantly increasing the pateint's intraocular pressure, which comprises: administering the composition of Emb-44.
Emb-49. The method of Emb-48, wherein the angiostatic steroid is selected from the group consisting of: tetrahydrocortisol; 21-Nor-5β-pregnan-3α,17α,20-triol; 4,9(11)-Pregnadien-17α,21-diol-3,20-dione; 4.9(11)-Pregnadien-17α,21-diol-3,20-dione-21-acetate.

## Claims

1. Use of a compound of the following formula: 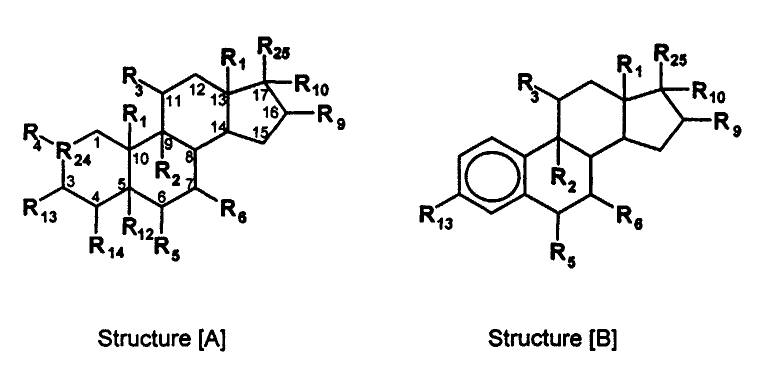 wherein
R₁ is H, β-CH₃ or β-C₂H₅;
R₂ is C₉-C₁₁ double bond;
R₃ is C₉-C₁₁ double bond;
R₄ is H, CH₃, Cl or F;
R₅ is H, OH, F, Cl, Br, CH₃, phenyl, vinyl or allyl;
R₆ is H or CH₃;
R₉ is CH₂CH₂OR₂₆, CH₂CH₂OC(=O)R₂₇, H, OH, CH₃, F, =CH₂, CH₂C(=O)OR₂₈, OR₂₆, O(C=O)R₂₇, or (C=O)CH₂(C=O)OR₂₆;
R₁₀ is -C≡CH, -CH=CH₂, halogen, CN, N₃, OR₂₆, OC(=O)R₂₇, H, OH, CH₃ or R₁₀ forms a second bond between positions C-16 and C-17;
R₁₂ is H or forms a double bond with R₁ or R₁₄;
R₁₃ is halogen, OR₂₆, OC(=O)R₂₇, NH₂, NHR₂₆, NHC(=O)R₂₇, N(R₂₆)₂, NC(=O)R₂₇, N₃, H, -OH, =O, -O-P(=O)(OH)₂, or -O-C(=O)-(CH₂)ₜCOOH where t is an integer from 2 to 6;
R₁₄ is H or forms a double bond with R₁₂;
R₂₄ = C, C₁-C₂ double bond, O;
R₂₅ = C(=O)CHR₂₈OC(=O)R₂₇; wherein
R₂₆ = C₁-C₆ (alkyl, branched alkyl, cycloalkyl, haloalkyl, aralkyl, aryl);
R₂₇ = R₂₆ + OR₂₆;
R₂₈ = H, C₁-C₆ (alkyl, branched alkyl, cycloalkyl),
for the preparation of a pharmaceutical composition for preventing and/or treating neovascularization.

2. Use according to claim 1, wherein the pharmaceutical composition is for preventing and/or treating ocular neovascularization.

3. Use according to claim 2, wherein the medicament is for preventing and/or treating retinal diseases including diabetic retinopathy, chronic glaucoma, retinal detachment, sickle cell retinopathy and senile macular degeneration due to subretinal neovascularization; rubeosis iritis; inflammatory diseases; chronic uveitis; neoplasms including retinoblastoma and pseudoglioma; Fuch's heterochromic iridocyclitis; neovascular glaucoma; corneal neovascularization including inflammatory, transplantation and developmental hypoplasia of the iris; neovascularization resulting following a combined vitrectomy and lensectomy; vascular diseases including retinal ischemia, choroidal vascular insufficiency, choroidal thrombosis and carotid artery ischemia; pterigium; neovascularization of the optic nerve; and neovascularization due to penetration of the eye or contusive ocular injury.
